(19) Europäisches Patentamt
European Patent Office
Office européen des brevets

(11) EP 0 452 500 B1

(12) EUROPEAN PATENT SPECIFICATION

(45) Date of publication and mention of the grant of the patent:
**12.03.1997 Bulletin 1997/11**

(21) Application number: **90915183.9**

(22) Date of filing: **19.10.1990**

(51) Int Cl.[6]: **C07D 417/12**, A01N 47/36, C07D 239/47, C07D 239/52, C07D 239/42, C07D 251/16

(86) International application number:
**PCT/JP90/01351**

(87) International publication number:
**WO 91/06546 (16.05.1991 Gazette 1991/11)**

# (54) SULFAMIDOSULFONYLUREA DERIVATIVES AND HERBICIDES

SULFAMIDOSULFONYLHARNSTOFFDERIVATE UND HERBIZIDE

DERIVES DE SULFAMIDOSULFONYLUREE ET HERBICIDES LES CONTENANT

(84) Designated Contracting States:
**AT BE CH DE DK ES FR GB GR IT LI LU NL SE**

(30) Priority: **27.10.1989 JP 281338/89**
**30.10.1989 JP 282764/89**
**04.12.1989 JP 314901/89**
**04.04.1990 JP 89629/90**

(43) Date of publication of application:
**23.10.1991 Bulletin 1991/43**

(73) Proprietor: **NISSAN CHEMICAL INDUSTRIES, LIMITED**
**Chiyoda-ku Tokyo 101 (JP)**

(72) Inventors:
- **MAKINO, Kenzi Nissan Chemical Industries Ltd. Funabashi-shi Chiba 274 (JP)**
- **MORIMOTO, K. Nissan Chemical Industries Ltd. Funabashi-shi Chiba 274 (JP)**
- **AKIYAMA, Shigeaki Nissan Chemical Industries Ltd. Funabashi-shi Chiba 274 (JP)**
- **SUZUKI, Hideaki Nissan Chemical Industries Ltd. Funabashi-shi Chiba 274 (JP)**
- **SUZUKI, K. Nissan Chem. Ind. Ltd. Seibutsukagaku Minamisaitama-gun Saitama 349-02 (JP)**
- **NAWAMAKI, T. Nissan Chem. Ind. Ltd. Seibustukagaku Minamisaitama-gun Saitama 349-02 (JP)**
- **WATANABE, S. Nissan Chem. Ind. Ltd Seibutsukagaku Minamisaitama-gun Saitama 349-02 (JP)**

(74) Representative:
**Wächtershäuser, Günter, Prof. Dr.**
**Patentanwalt,**
**Tal 29**
**80331 München (DE)**

(56) References cited:
**EP-A- 0 131 258** **EP-A- 0 319 689**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 452 500 B1

## Description

TECHNICAL FIELD

The present invention relates to novel sulfamidosulfonylurea derivatives and herbicides containing them as active ingredients.

BACKGROUND TECHNIQUE

It is indispensable to use herbicides to protect important crop plants such as rice, wheat, corn, soybean, cotton and sugar beet from weeds and thereby to increase the harvest. Especially in recent years, a selective herbicide is desired which is capable of selectively killing weeds without showing any phytotoxicity against crop plants when applied to the foliages of crop plants and weeds simultaneously in a field where such useful crop plants and weeds are coexistent. Further, with a view to avoiding environmental pollution and reducing the costs for transportation and application, researches and developments have been conducted for many years for compounds having high herbicidal effects at low doses. Some of the compounds having such properties are presently used as selective herbicides. However, there still exists a need for better compounds having such properties.

As the prior art showing a chemical structure similar to that of the compounds of the present invention, Japanese Unexamined Patent Publications No. 103371/1983, No. 48973/1985 and No. 151577/1989 disclose compounds having a sulfonylurea bonded to a nitrogen atom.

Japanese Unexamined Patent Publications No. 48973/1985 and No. 151577/1989 disclose compounds having a sulfonylurea bonded to the nitrogen atom of a sulfonamide structure.

EP-A-0409114 discloses certain sulfamidosulfonylurea derivatives for use as herbicides and growth regulating agents in plants. EP-A-0409114 does not disclose sulfamidosulfonylurea derivatives wherein an oxygen atom is bound to the nitrogen atom bridging the sulfur atoms.

However, the compounds of the present invention wherein a sulfonylurea is bonded to the nitrogen atom of a special sulfamide structure, have not been known at all, and they are novel compounds.

DISCLOSURE OF THE INVENTION

The present inventors have conducted extensive researches over years to develop selective herbicides for important crop plants and have studied herbicidal properties of many compounds with an aim to find out compounds having higher herbicidal activities as well as selectivity. As a result, it has been found that sulfamidosulfonylurea derivatives of the following formula (I) and agriculturally suitable salts thereof (hereinafter referred to as the compounds of the present invention) exhibit remarkably strong herbicidal activities against many weeds in soil treatment or in foliage treatment and at the same time have a high level of safety for important crop plants such as wheat, corn, cotton, soybean, sugar beet and rice. The present invention has been accomplished on the basis of this discovery. On the other hand, since the compounds of the present invention show high herbicidal activities at a very low dose as compared with conventional herbicides, they are also useful as herbicides for orchards or for non-agricultural fields.

Namely, the present invention provides a sulfamidosulfonylurea derivative of the formula (1) or an agriculturally suitable salt thereof:

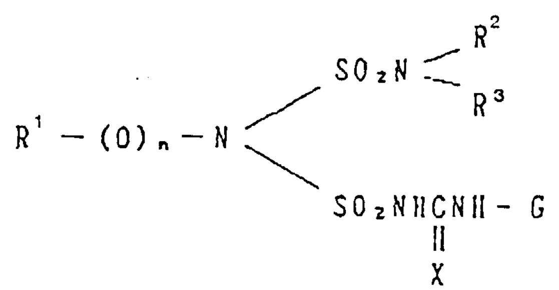

(I)

wherein $R^1$ is a hydrogen atom, a $C_1$-$C_6$ lower alkyl group, a $C_3$-$C_7$ cycloalkyl group, a $C_2$-$C_6$ lower alkenyl group, a $C_2$-$C_6$ lower alkynyl group, a $C_1$-$C_6$ lower alkyl group substituted by a $C_1$-$C_6$ lower alkoxy group, a $C_1$-$C_6$ lower alkyl group substiuted by a $C_1$-$C_6$ lower mono- or polyhalogenoalkoxy group, a $C_1$-$C_6$ lower alkyl group substituted by a $C_1$-$C_6$ lower alkylthio group, a $C_1$-$C_6$ lower alkyl group substituted by a $C_1$-$C_6$ lower alkylsulfonyl group, a $C_1$-$C_6$ lower mono- or polyhalogenoalkyl group, a $C_1$-$C_6$ lower alkyl group substituted by a cyano group, a $C_1$-$C_6$ lower alkyl group

substituted by a $C_1$-$C_6$ lower alkoxycarbonyl group, a $C_1$-$C_6$ lower alkyl group substituted by a $C_1$-$C_6$ lower alkylcarbonyl group, a benzyl group (provided that such a benzyl group may be substituted by a halogen atom, a trifluoromethyl group, a $C_1$-$C_6$ lower alkyl group, a $C_1$-$C_6$ lower alkoxy group or a $C_1$-$C_6$ lower alkoxycarbonyl group), $R^2$ is a hydrogen atom or a $C_1$-$C_6$ lower alkyl group, $R^3$ is a $C_1$-$C_6$ lower alkyl group, a phenyl group or a benzyl group (provided that such a phenyl group or a benzyl group may be substituted by a halogen atom, a trifluoromethyl group, a $C_1$-$C_6$ lower alkyl group, a $C_1$-$C_6$ lower alkoxy group, a $C_1$-$C_6$ lower alkoxycarbonyl group or a nitro group), or $R^2$ and $R^3$ form, together with the nitrogen atom to which they are bonded, a saturated 5-7 membered heterocyclic group;
n is 1;
X is an oxygen atom or a sulfur atom; and
G is

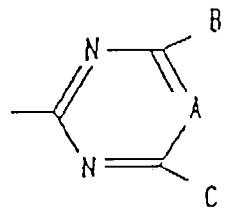

wherein A is a CH group or a nitrogen atom, and each of B and C independently is a $C_1$-$C_4$ lower alkyl group, a $C_1$-$C_4$ lower alkoxy group, a $C_1$-$C_4$ lower halogenoalkyl group, a $C_1$-$C_4$ lower halogenoalkoxy group, a halogen atom or a $C_1$-$C_4$ lower mono-alkylamino group.

The present invention also provides a selective herbicide containing one or more compounds of the present invention as active ingredients.

Further, the present invention provides a herbicidal composition comprising one or more compounds of the present invention and one member selected from the group consisting of alachlor, acetochlor, metolachlor, primisulfuron and nicosulfuron, as active ingredients.

Now, the present invention will be described in detail with reference to the preferred embodiments.

The compounds of the formula (I) of the present invention can easily be prepared by any one of the following reaction schemes 1 to 6.

Reaction scheme 1

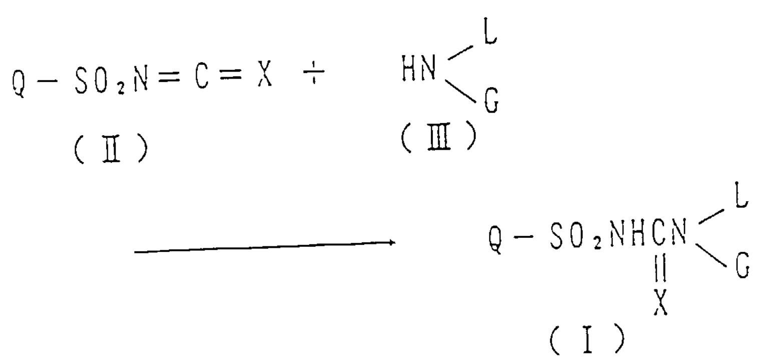
(II) (III) (I)

wherein Q is

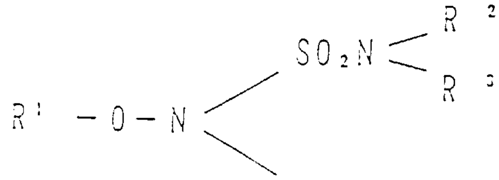

In the above formulas, $R^1$, $R^2$, $R^3$, G and X are as defined above, and L is hydrogen.

Namely, a sulfamidosulfonyliso(thio)cyanate derivative (II) is dissolved in a sufficiently dried inert solvent such as dioxane, acetonitrile or acetone, then pyrimidine or a triazine derivative of the formula (III) is added thereto, and the mixture is stirred, whereby the reaction usually proceeds swiftly and the compound (I) of the present invention is obtained. When the reaction hardly proceeds, a very small amount of a suitable base such as triethylamine, triethylenediamine, pyridine, sodium methoxide, sodium hydride or potassium carbonate may be added, whereby the reaction readily proceeds.

Reaction scheme 2

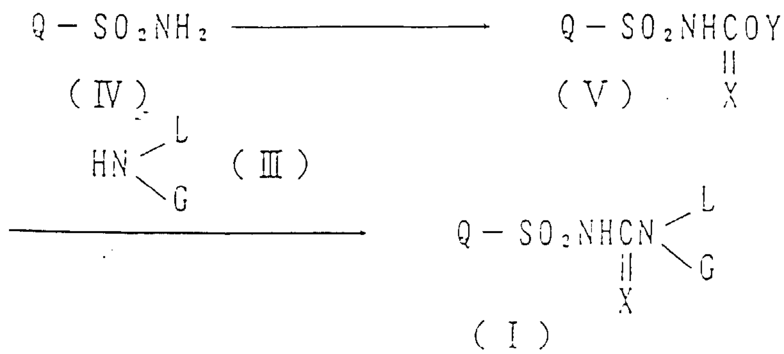

wherein Q is

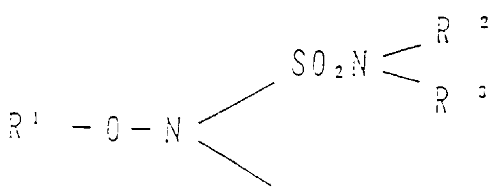

In the above formulas, $R^1$, $R^2$, $R^3$, G, and X are as defined above, Y is a $C_1$-$C_6$ alkyl group or a phenyl group, and L is hydrogen.

Namely, a sulfamidosulfonamide derivative (IV) is reacted with chloro(thio)formic acid ester or (thio)carbonic acid di-ester in a solvent such as acetone, methyl ethyl ketone or acetonitrile in the presence of a base such as potassium carbonate to obtain a compound (V). Then, it is heated together with a compound (III) in a solvent such as toluene to obtain the compound (I) of the present invention.

Reaction Scheme 3

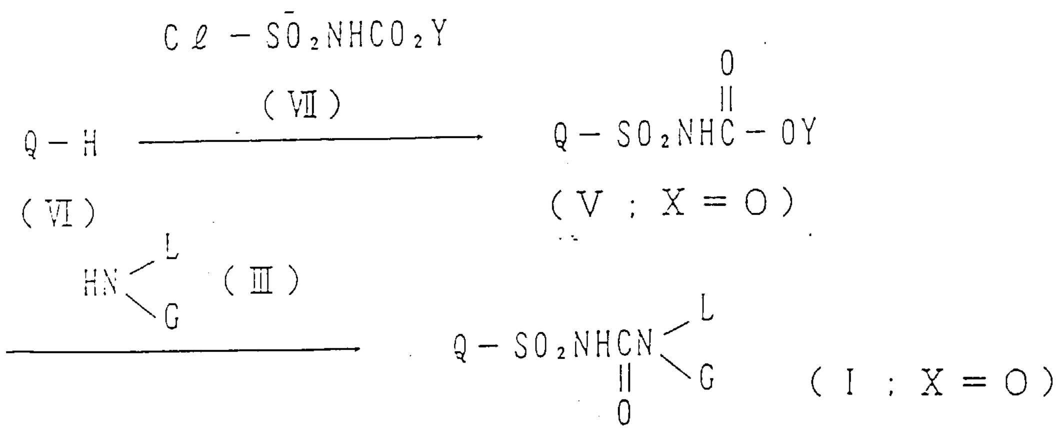

wherein Q is

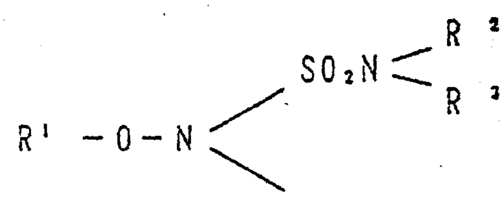

In the above formulas, $R^1$, $R^2$, $R^3$, G and Y are as defined above, and L is hydrogen.

The reaction of a sulfamide (VI) with phenyl N-chlorosulfonylcarbamate (VII; Y = phenyl group) or an alkyl N-chlorosulfonylcarbamate (VII; Y = $C_1$-$C_6$ alkyl group) is conducted usually by employing from 0.5 to 3.0 mol, preferably from 0.9 to 1.2 mol, of the carbamate derivative (VII) per mol of the sulfamide (VI).

The reaction temperature may optionally be selected within a range of from -50°C to 100°C, preferably from -20°C to 30°C.

This reaction is conducted by means of various bases. The base is used in an amount of from 0.5 to 4.0 mol per mol of the sulfamide (VI).

Suitable bases include metal hydrides such as sodium hydride, metal alkoxides such as sodium ethoxide, alkyl metals such as n-butyl lithium, organic bases such as triethylamine, pyridine and 1,8-diazabicyclo[5,4,0]-7-undecene (DBU), and inorganic bases such as potassium hydroxide and sodium hydroxide. It is particularly preferred to employ a metal hydride.

A suitable solvent for this reaction is a solvent inert to the reaction, which includes an aromatic hydrocarbon such as benzene, toluene or xylene, a halogenated hydrocarbon such as dichloromethane, chloroform or carbon tetrachloride, an ether such as ethyl ether, isopropyl ether, dioxane or tetrahydrofuran, a nitrile such as acetonitrile or propionitrile, a hydrocarbon such as petroleum ether, petroleum benzin or hexane, a ketone such as acetone or methyl ethyl ketone, an ester such as ethyl acetate, and an amide such as N,N-dimethylformamide, N,N-dimethylacetamide or N,N,N',N'-hexamethylphosphoric acid triamide. These solvents may be used alone or in combination as a mixture. It is particularly preferred to employ an ether or an amide.

Then, phenyl N-sulfamidosulfonylcarbamate (V; X = O, Y = phenyl group) or an alkyl N-sulfamidosulfonylcarbamate (V; X = O, Y is lower alkyl group) and the compound (III) are heated in an solvent such as benzene or toluene to obtain the compound of the present invention (I; X = O).

Reaction scheme 4

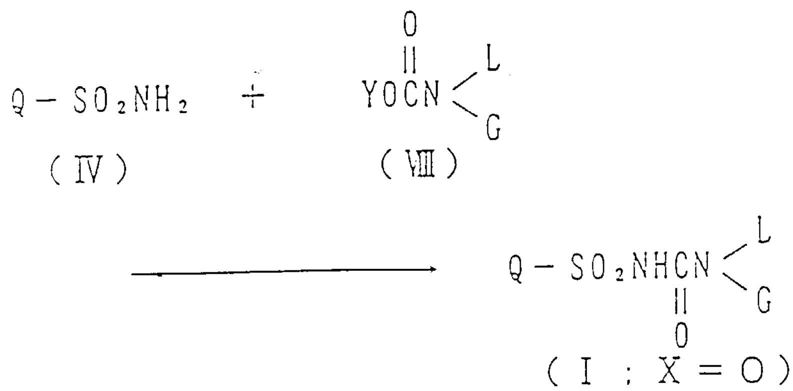

wherein Q is

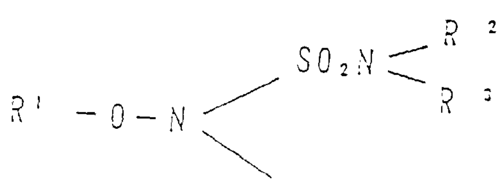

In the above formulas, $R^1$, $R^2$, $R^3$, G and Y are as defined above, and L is hydrogen.

Namely, a sulfamidosulfonamide derivative (IV) is reacted with a carbamate derivative (VIII) in a solvent such as acetone, acetonitrile or dioxane in the presence of an inorganic base such as potassium carbonate, or an organic base such as triethylamine or DBU, to obtain the compound of the present invention (I; X = O).

Reaction scheme 5

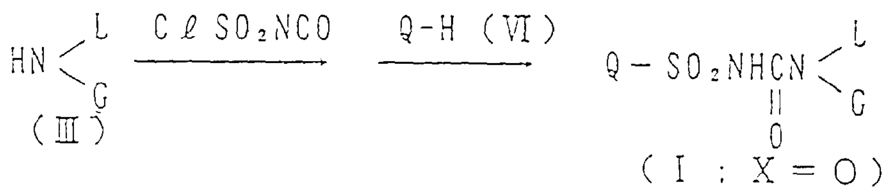

(III)
(I : X = O)

wherein Q is

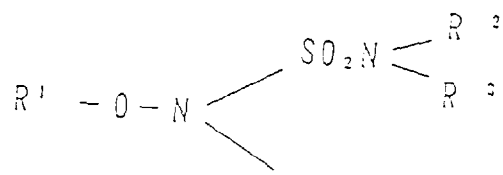

In the above formulas, $R^1$, $R^2$, $R^3$ and G are as defined above, and L is hydrogen.

Namely, an amine (III) is reacted with chlorosulfonylisocyanate in a solvent such as tetrahydrofuran, dimethoxyethane, acetonitrile, propionitrile, N,N-dimethylformamide, dichloromethane, dichloroethane, benzene or toluene and then reacted with a sulfamide (VI) in the presence of a base such as triethylamine, pyridine, sodium hydride, sodium methoxide, sodium ethoxide, sodium hydroxide, potassium hydroxide or potassium carbonate, to obtain the compound of the present invention (I; X=O).

Reaction scheme 6

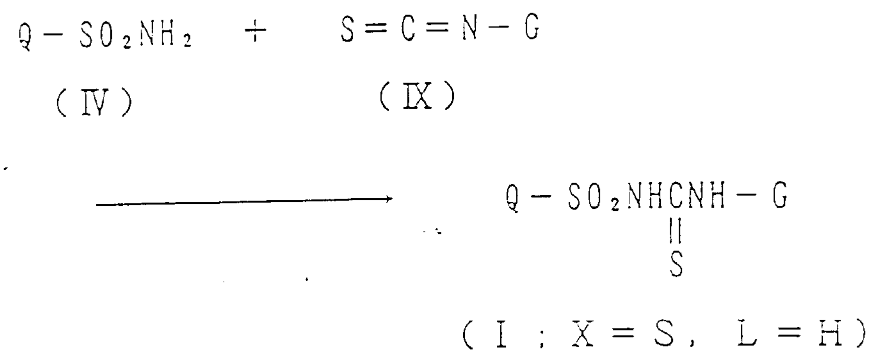
(IV)
(IX)
(I ; X = S, L = H)

wherein Q is

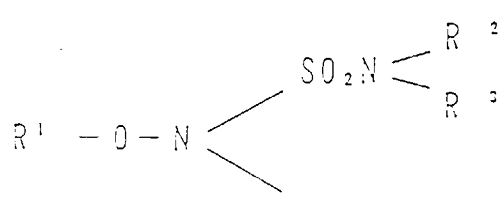

In the above formulas, G is as defined above.

Namely, a sulfamidosulfonamide derivative (IV) is reacted with an isothiocyanate derivative (IX) in a solvent such as acetone, acetonitrile or dioxane in the presence of an inorganic base such as potassium carbonate, or an organic base such as triethylamine or DBU to obtain the compound of the present invention (I; X = S, L = H).

The sulfamidosulfonyliso(thio)cyanate derivative (II) to be used as a starting material in reaction scheme 1 can be synthesized from a sulfamidosulfonamide derivative (IV) in accordance with the methods disclosed in e.g. Japanese Unexamined Patent Publications No. 148879/1983, No. 31775/1984 and No. 13266/1980.

Further, the sulfamidosulfonylisocyanate (II; X = O) can be synthesized also by the method of reaction scheme 7 in accordance with the method disclosed in e.g. Japanese Unexamined Patent Publication No. 81320/1974.

Reaction scheme 7

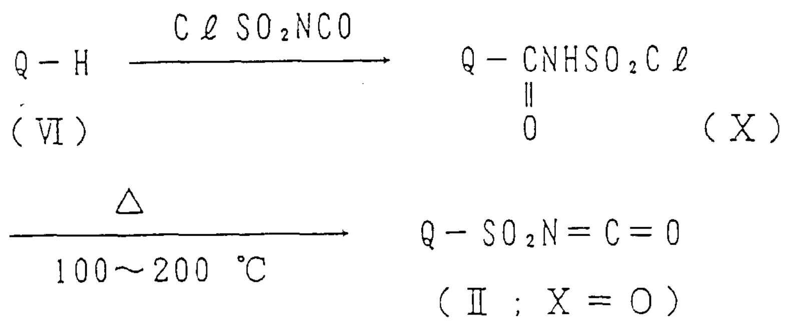

(VI) (X) (II ; X = O)

In the above formulas, Q is as defined above.

The sulfamidosulfonyliso(thio)cyanate derivative (II), the sulfamidosulfonamide derivative (IV), the phenyl N-sulfamidosulfonyl(thio)carbamate (V; Y = phenyl group) and the alkyl N-sulfamidosulfonyl(thio)carbamate (V; Y = lower alkyl group) used as intermediates in the present invention are also novel compounds. The sulfamidosulfonamide derivative (IV) can be synthesized from a sulfamide derivative (VI) by the methods of reaction schemes 8 and 9.

Reaction scheme 8

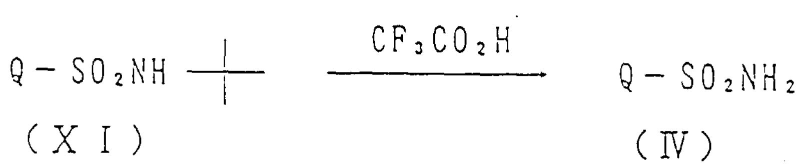

(X I) (IV)

In the above formulas, Q is as defined above.

In the reaction scheme 8, the removal of the tert-butyl group is conducted using trifluoroacetic acid. The amount of the trifluoroacetic acid may optionally be selected within a range of from an equimolar amount to an excess amount. The trifluoroacetic acid may also be used as a solvent without any problem.

The reaction temperature may optionally be selected within a range of from -50°C to 80°C, preferably from -20°C to 30°C.

When a solvent is employed for this reaction, it is a solvent inert to the reaction, which includes an aromatic hydrocarbon such as benzene, toluene or xylene, a halogenated hydrocarbon such as dichloromethane, chloroform or carbon tetrachloride, an ether such as ethyl ether, isopropyl ether, dioxane or tetrahydrofuran, a nitrile such as acetonitrile or propionitrile, a hydrocarbon such as petroleum ether, petroleum benzin or hexane, a ketone such as acetone or methyl ethyl ketone, an ester such as ethyl acetate, and an amide such as N,N-dimethylformamide, N,N-dimethylacetamide or N,N,N',N'-hexamethylphosphoric acid triamide. These solvents may be used alone or in combination as a mixture.

Reaction scheme 9

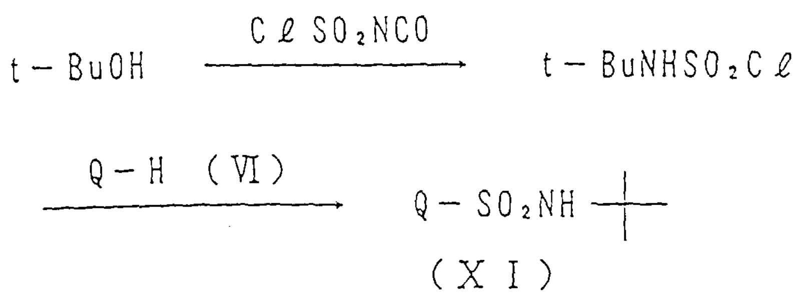


In the above formulas, Q is as defined above.

In reaction scheme 9, the reaction of t-butanol with chlorosulfonylisocyanate can be conducted by a method per se known in accordance with e.g. Japanese Unexamined Patent Publication No. 101323/1975.

The reaction of the sulfamide (VI) with tert-butylsulfamoyl chloride is conducted using from 0.5 to 3.0 mol, preferably from 0.9 to 1.2 mol, of tert-butylsulfamoyl chloride per mol of the sulfamide (VI).

The reaction temperature may optionally be selected within a range of from -50°C to 100°C, preferably from -20°C to 30°C.

The reaction may be conducted using various bases. The base is used in an amount of from 0.5 to 4.0 mol, preferably from 0.8 to 2.2 mol, per mol of the sulfamide (VI). Suitable bases include a metal hydride such as sodium hydride, a metal alkoxide such as sodium ethoxide, an alkyl metal such as n-butyl lithium, an organic base such as triethylamine, pyridine, 1,8-diazabicyclo[5,4,0]-7-undecene (DBU), and an inorganic base such as potassium hydroxide or sodium hydroxide. It is particularly preferred to employ a metal hydride.

A suitable solvent for the reaction is a solvent inert to the reaction, which includes an aromatic hydrocarbon such as benzene, toluene or xylene, a halogenated hydrocarbon such as dichloromethane, chloroform or carbon tetrachloride, an ether such as ethyl ether, isopropyl ether, dioxane or tetrahydrofuran, a nitrile such as acetonitrile or propionitrile, a hydrocarbon such as petroleum ether, petroleum benzin or hexane, a ketone such as acetone or methyl ethyl ketone, an ester such as ethyl acetate, and an amide such as N,N-dimethylformamide, N,N-dimethylacetamide or N,N,N',N'-hexamethylphosphoric acid triamide. These solvents may be used alone or in combination as a mixture. It is particularly preferred to employ an ether or an amide.

In the reaction scheme 3, the phenyl N-chlorosulfonylcarbamate (VII; Y = phenyl group) and the alkyl N-chlorosulfonylcarbamate (VII; Y = lower alkyl group) can be prepared by methods per se known in accordance with e.g. Chemische Berichte, vol. 96, p. 56 (1963). The sulfamide (VI) used as the starting material of the above reaction can be prepared in accordance with e.g. Journal of the American Chemical Society, vol. 66, p. 1242 (1944), U.S. Patent 2,826,594, Journal of Synthetic Organic Chemistry, Japan, vol, 27 (No. 10), p. 980 (1969), U.S. Patent 2, 624,729, Chemische Berichte, vol. 111, p. 1915 (1978), Japanese Unexamined Patent Publications No. 79894/1978 and No. 208289/1983, Indian Journal of Chemistry, Section B, vol. 21B, p. 941 (1982). As representative examples, syntheses of N,N-dimethyl-N'-methoxysulfamide, and N,N-dimethyl-N'-trifluoromethylthiosulfamide will be given as reaction schemes 10 to 11.

Reaction scheme 10

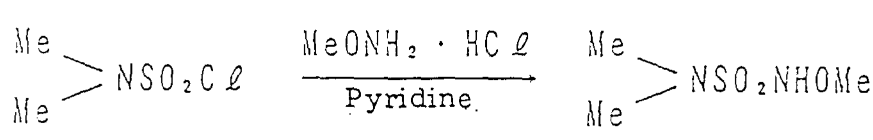

Reaction scheme 11

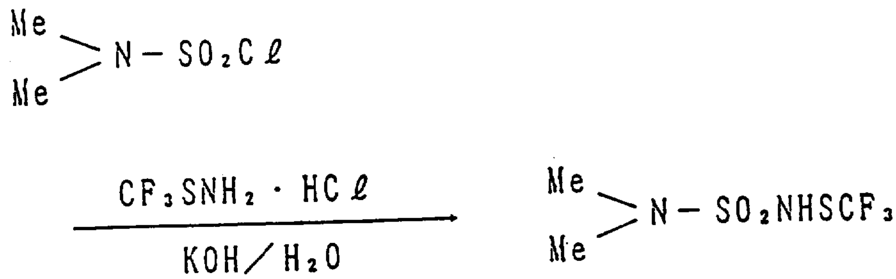


Now, the syntheses of the compounds of the present invention will be described as Examples. However, it should be understood that the present invention is by no means restricted to such specific Examples.

EXAMPLE 1

Preparation of 1-[(N-dimethylsulfamoyl-N-methoxyamino)sulfonyl]-3-(4,6-dimethoxypyrimidin-2-yl)urea

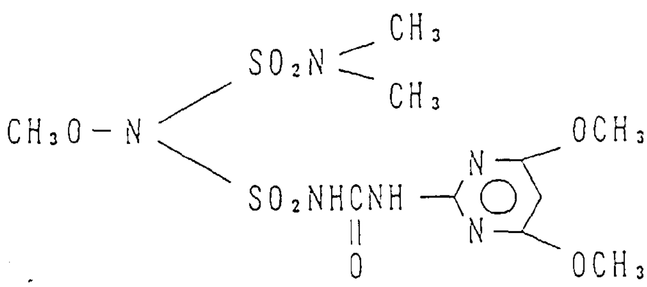

(Compound No. 1)

To 100 mℓ of dry tetrahydrofuran (THF) containing 1.87 g (42.9 mmol) of 55% sodium hydride, 3.0 g (19.5 mmol) of N,N-dimethyl-N'-methoxysulfamide dissolved in 20 mℓ of dry THF was added under cooling with ice, and the mixture was continuously stirred at the same temperature for 10 minutes.

Then, 50 mℓ of dry tetrahydrofuran (THF) containing 4.83 g (20.5 mmol) of phenyl N-chlorosulfonylcarbamate was dropwise added thereto, and the mixture was gradually heated to room temperature and continuously stirred at room temperature for one hour.

The reaction mixture was poured into 500 mℓ of ice water containing 5 g of 35% hydrochloric acid and extracted with diethyl ether. The diethyl ether layer was sequentially washed with water and a saturated sodium chloride aqueous solution and then dried over anhydrous magnesium sulfate. Then, the solvent was distilled off under reduced pressure to obtain 5.4 g of phenyl N-[(N-dimethylsulfamoyl-N-methoxyamino)sulfonyl]carbamate as an orange-colored oil.

Then, a mixture comprising 3 g (8.5 mmol) of the above compound, 0.93 g (6 mmol) of 2-amino-4,6-dimethoxypyrimidine and 20 mℓ of dry benzene was refluxed under heating for 10 minutes. After cooling, the solvent was distilled off under reduced pressure. The residue was washed with diethyl ether and collected by filtration to obtain 1.0 g of desired 1-[(N-dimethylsulfamoyl-N-methoxyamino)sulfonyl]-3-(4,6-dimethoxypyrimidin-2-yl)urea. Melting point 166 - 167°C

EXAMPLE 2 (REFERENCE)

Preparation of 1-[(N-N-methyl-N-methoxyaminosulfonyl)-N-ethylamino)sulfonyl]-3-(4,6-dimethoxypyrimidin-2-yl)urea

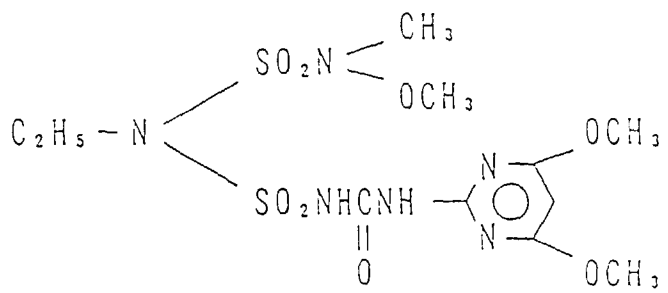

To 50 mℓ of dry tetrahydrofuran (THF) containing 1.60 g (36.7 mmol) of 55% sodium hydride, 2.8 g (16.7 mmol) of N-methyl-N-methoxy-N'-ethylsulfamide dissolved in 20 mℓ of dry THF was added under cooling with ice and continuously stirred at the same temperature for 10 minutes.

Then, 30 mℓ of dry THF containing 4.13 g (17.5 mmol) of phenyl N-chlorosulfonylcarbamate was dropwise added, and the mixture was gradually heated to room temperature and continuously stirred at room temperature for one hour.

The reaction mixture was poured into 500 mℓ of ice water containing 4 g of 35% hydrochloric acid and extracted with diethyl ether. The diethyl ether layer was sequentially washed with water and a saturated sodium chloride aqueous solution and then dried over anhydrous magnesium sulfate. Then, the solvent was distilled off under reduced pressure to obtain 5.0 g of phenyl N-[(N-(N-methyl-N-methoxyaminosulfonyl)-N-ethylamino)sulfonyl]carbamate as an orange-colored oil.

Then, a mixture comprising 2.5 g (6.8 mmol) of the above compound, 0.74 g (4.8 mmol) of 2-amino-4,6-dimethoxypyrimidine and 50 mℓ of dry benzene, was refluxed under heating for 10 minutes. After cooling the mixture, the solvent was distilled off under reduced pressure. The residue was washed with diethyl ether and collected by filtration to obtain 1.2 g of desired 1-[(N-(N-methyl-N-methoxyaminosulfonyl)-N-ethylamino)sulfonyl]-3-(4,6-dimethoxypyrimidin-2-yl)urea.

Melting point: 157 - 158°C.

EXAMPLE 3 (REFERENCE)

Preparation of 1-(2-methoxy-1,2,5-thiadiazolidine-1,1-dioxide-5-sulfonyl)-3-(4,6-dimethoxypyrimidin-2-yl)urea

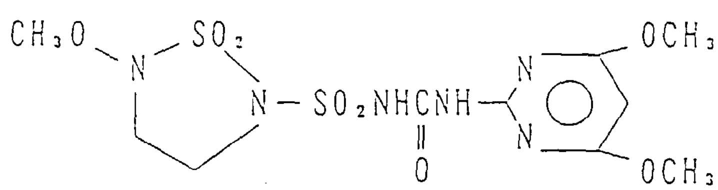

To 15 mℓ of dry tetrahydrofuran (THF) containing 0.25 g (6.25 mmol) of 60% sodium hydride, 0.43 g (2.83 mmol) of 2-methoxy-1,2,5-thiadiazolidine-1,1-dioxide dissolved in 5 mℓ of dry THF was added under cooling with ice, and the mixture was stirred under a nitrogen stream at room temperature for 14 hours.

Then, the system was cooled to 0°C, and 10 mℓ of dry THF containing 0.7 g (3 mmol) of phenyl N-chlorosulfonylcarbamate was dropwise added thereto. The mixture was gradually heated to room temperature and continuously stirred at room temperature for one hour.

The reaction mixture was poured into 100 mℓ of ice water containing 0.6 g of 35% hydrochloric acid and extracted with diethyl ether. The diethyl ether layer was sequentially washed with water and a saturated sodium chloride aqueous solution and then dried over anhydrous magnesium sulfate. Then, the solvent was distilled off under reduced pressure to obtain 0.7 g of desired phenyl N-(2-methoxy-1,2,5-thiadiazolidine-1,1-dioxide-5-sulfonyl)carbamate as a yellow viscous oil.

Then, a mixture comprising 0.7 g (2 mmol) of the above compound, 0.31 g (2 mmol) of 2-amino-4,6-dimethoxypyrimidine and 10 mℓ of dry benzene, was refluxed under heating for 10 minutes. After cooling the mixture, the solvent was distilled off under reduced pressure and the residue thereby obtained was washed with diethyl ether to obtain 0.5

g of desired 1-(2-methoxy-1,2,5-thiadiazolidine-1,1-dioxide-5-sulfonyl)-3-(4,6-dimethoxypyrimidin-2-yl)urea. Melting point: 162 - 163°C

The structures and physical properties of the compounds prepared in the same manner as in the preceding Examples, are shown below:

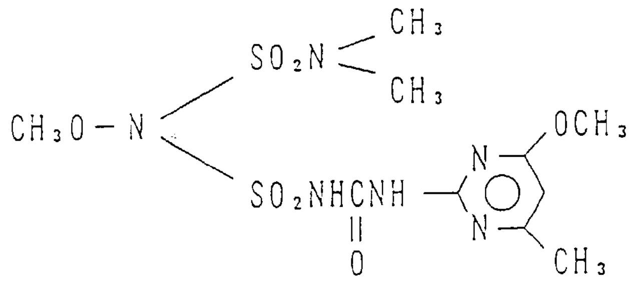

Compound No. 2　　m.p. 148 ~ 150 ℃

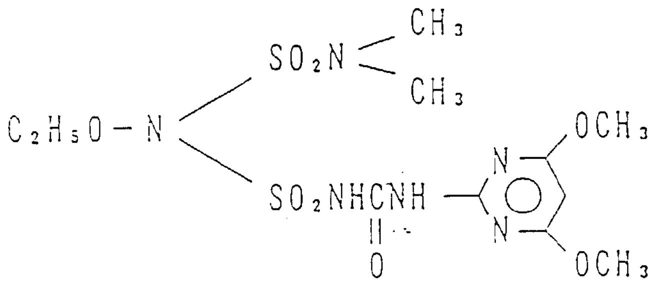

Compound No. 3　　m.p. 150 ~ 151 ℃

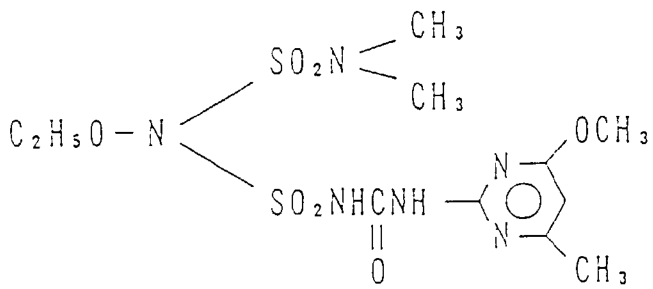

Compound No. 4　　m.p. 154 ~ 155 ℃

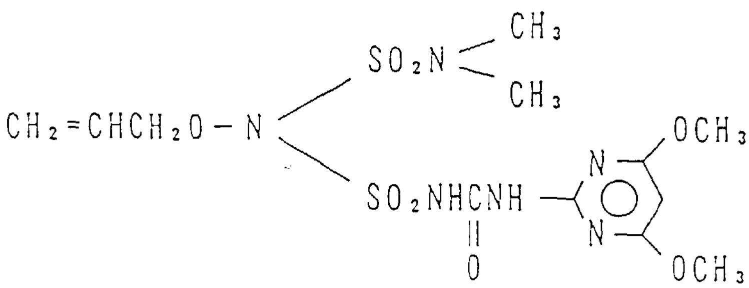

Compound No. 5　　m.p. 144 ~ 145 ℃

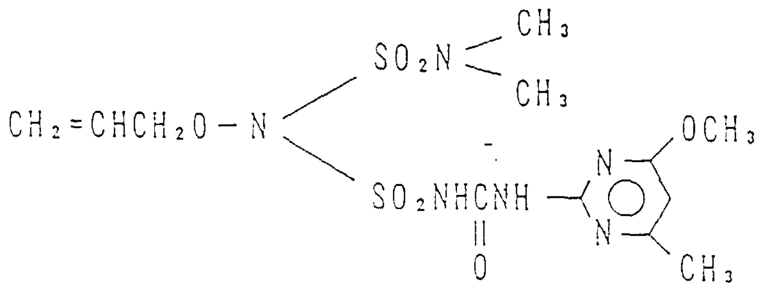

Compound No. 6 m.p. 138 ~ 139 ℃

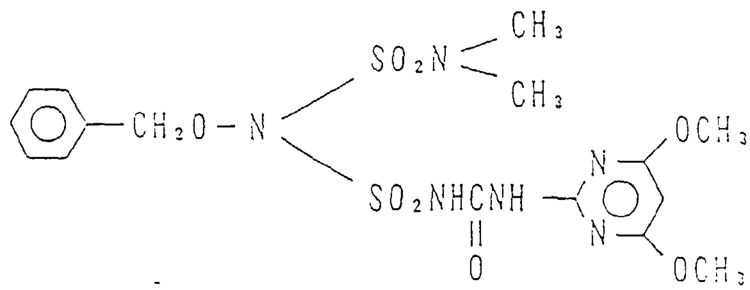

Compound No. 7 m.p. 127 ~ 128 ℃

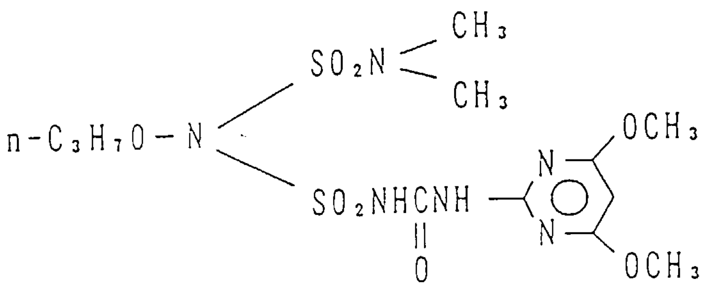

Compound No. 11 m.p. 127 ~ 128 ℃

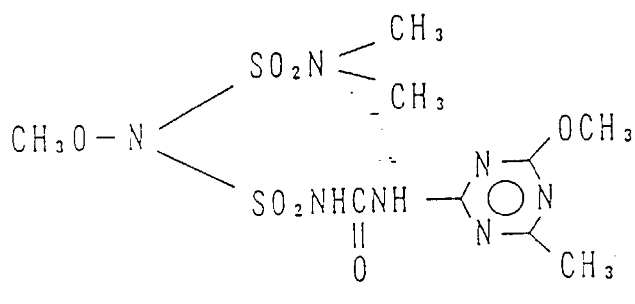

Compound No. 12 m.p. 118 ~ 120 ℃

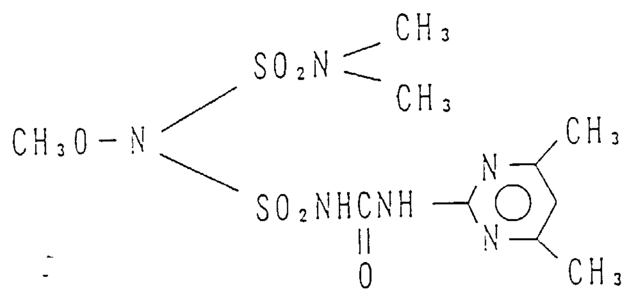

Compound No. 13 m.p. 165～166 ℃

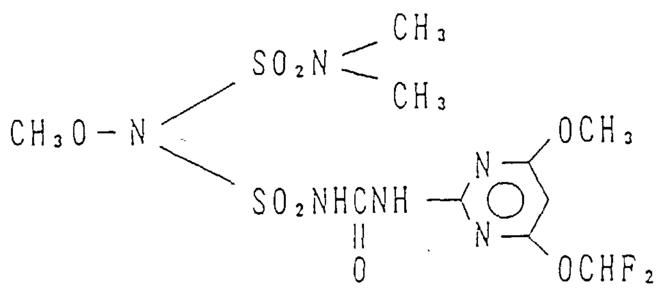

Compound No. 14 m.p. 137～139 ℃

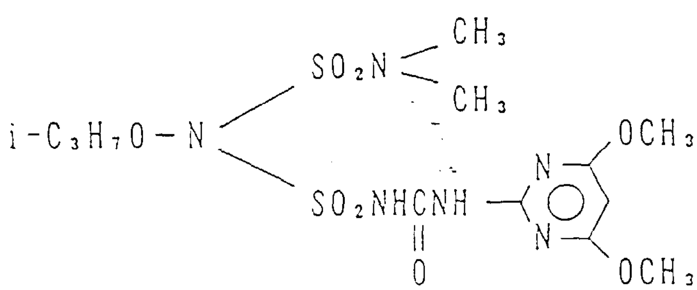

Compound No. 15 m.p. 140～141 ℃

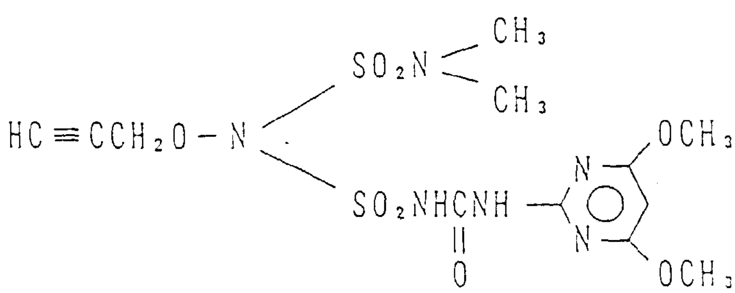

Compound No. 16 m.p. 150～151 ℃

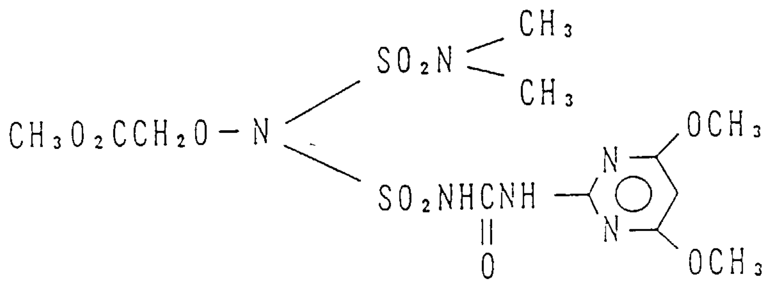

Compound No. 17 m.p. 141 ~ 142 ℃

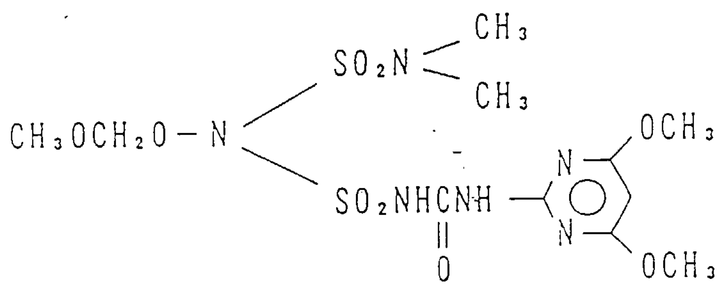

Compound No. 18 m.p. 147 ~ 148 ℃

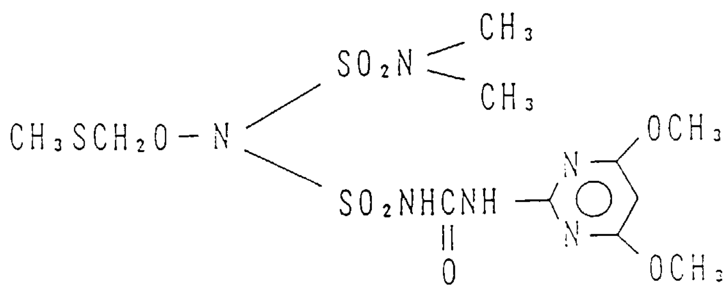

Compound No. 19 m.p. 117 ~ 118 ℃

Now, Tables 1 to 26 present examples of specific compounds of the present invention including the compounds prepared in the preceding Examples. However, it should be understood that the compounds of the present invention are not limited to such specific examples.

Symbols used in the Tables have the following meanings:

Me: methyl group, Et: ethyl group, Pr-n: propyl group,
Pr-i: isopropyl group, Pr-c: cyclopropyl group,
Bu-n: n-butyl group, Bu-s: sec-butyl group, Bu-i:
isobutyl group, Bu-t, tert-butyl group, Pen-n: n-pentyl group, Ph: phenyl group, and Gn has the same meaning as above-mentioned G and includes Ga, Gb, Gc as defined below.

Ga = G1-G13 (i.e. it represents all of G1 to G13).
Gb = G1-G6 (i.e. it represents all of G1 to G6).
Gc = G1-G3 (i.e. it represents all of G1 to G3).

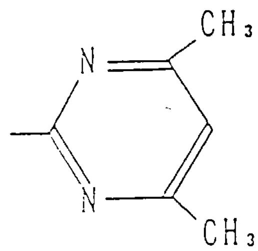

G1

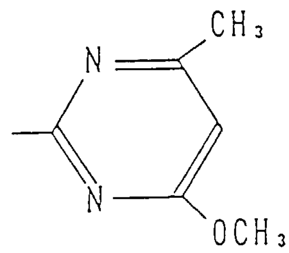

G2

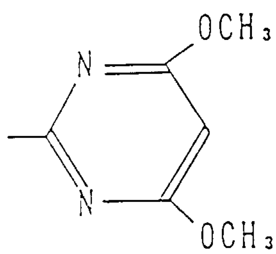

G3

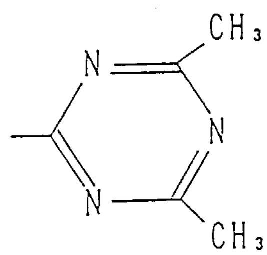

G4

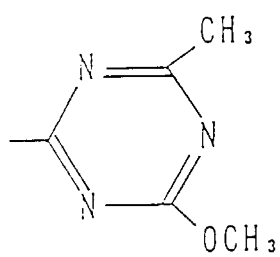

G5

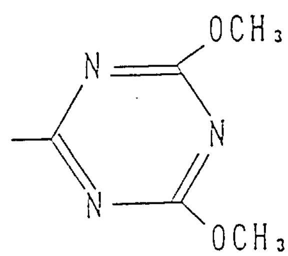

G6

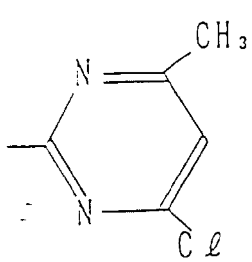

G7

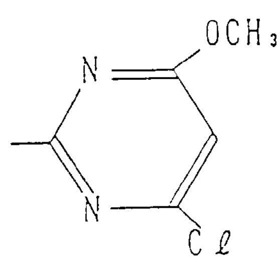

G8

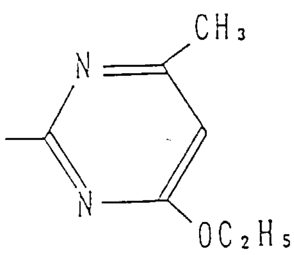

G9

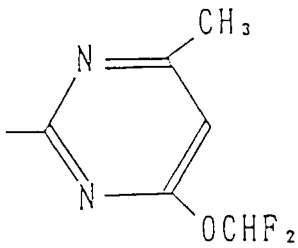

G10

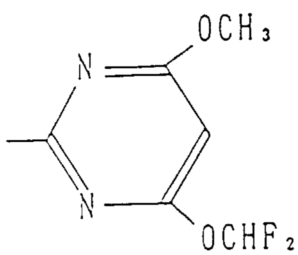

G11

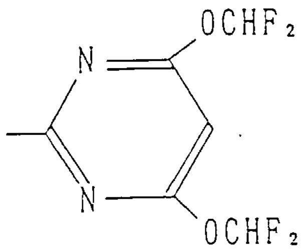

G12

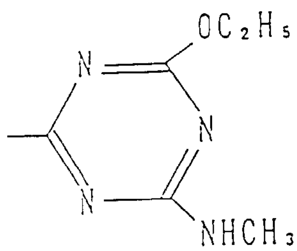

G13

Table 1

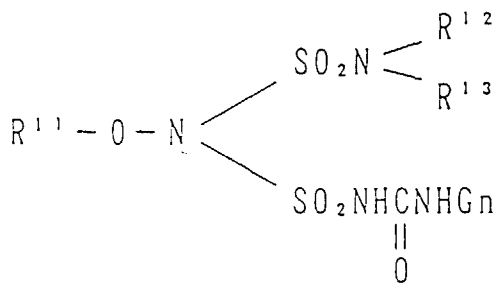

| $R^{11}$ | $R^{12}$ | $R^{13}$ | Gn |
|---|---|---|---|
| Me | H | Me | Gc |
| Me | H | Et | Gc |
| Me | Me | Me | Ga |
| Me | Me | Et | Ga |
| Me | Me | Pr-n | Gb |
| Me | Me | Pr-i | Gb |
| Me | Me | Bu-n | Gb |
| Me | Me | Ph | Ga |
| Me | Me | $Ph-CH_2$ | Ga |
| Me | Et | Et | Gb |
| Me | Pr-n | Pr-n | Gb |
| Me | $-(CH_2)_4-$ | | Ga |
| Me | $-(CH_2)_5-$ | | Gb |
| Et | H | Me | Gc |
| Et | H | Et | Gc |
| Et | Me | Me | Ga |
| Et | Me | Et | Ga |
| Et | Me | Pr-n | Gb |

Table 1 (continued)

| $R^{11}$ | $R^{12}$ | $R^{13}$ | Gn |
|---|---|---|---|
| Et | Me | Pr-i | Gb |
| Et | Me | Bu-n | Gb |
| Et | Me | Ph | Ga |
| Et | Me | $Ph-CH_2$ | Ga |
| Et | Et | Et | Gb |
| Et | Pr-n | Pr-n | Gb |
| Et | $-(CH_2)_4-$ | | Ga |
| Et | $-(CH_2)_5-$ | | Gb |
| Pr-n | H | Me | Gc |
| Pr-n | H | Et | Gc |
| Pr-n | Me | Me | Ga |
| Pr-n | Me | Et | Ga |
| Pr-n | Me | Pr-n | Gb |
| Pr-n | Me | Pr-i | Gb |
| Pr-n | Me | Bu-n | Gb |
| Pr-n | Me | Ph | Ga |
| Pr-n | Me | $Ph-CH_2$ | Ga |
| Pr-n | Et | Et | Gb |
| Pr-n | Pr-n | Pr-n | Gb |
| Pr-n | $-(CH_2)_4-$ | | Ga |
| Pr-n | $-(CH_2)_5-$ | | Gb |
| Pr-c | H | Me | Gc |
| Pr-c | H | Et | Gc |
| Pr-c | Me | Me | Ga |
| Pr-c | Me | Et | Ga |

Table 1 (continued)

| $R^{11}$ | $R^{12}$ | $R^{13}$ | Gn |
|---|---|---|---|
| Pr-c | Me | Pr-n | Gb |
| Pr-c | Me | Pr-i | Gb |
| Pr-c | Me | Bu-n | Gb |
| Pr-c | Me | Ph | Ga |
| Pr-c | Me | Ph-$CH_2$ | Ga |
| Pr-c | Et | Et | Gb |
| Pr-c | Pr-n | Pr-n | Gb |
| Pr-c | $-(CH_2)_4-$ | | Ga |
| Pr-c | $-(CH_2)_5-$ | | Gb |
| $CH_2$=CH | H | Me | Gc |
| $CH_2$=CH | H | Et | Gc |
| $CH_2$=CH | Me | Me | Ga |
| $CH_2$=CH | Me | Et | Ga |
| $CH_2$=CH | Me | Pr-n | Gb |
| $CH_2$=CH | Me | Pr-i | Gb |
| $CH_2$=CH | Me | Bu-n | Gb |
| $CH_2$=CH | Me | Ph | Ga |
| $CH_2$=CH | Me | Ph-$CH_2$ | Ga |
| $CH_2$=CH | Et | Et | Gb |
| $CH_2$=CH | Pr-n | Pr-n | Gb |
| $CH_2$=CH | $-(CH_2)_4-$ | | Ga |
| $CH_2$=CH | $-(CH_2)_5-$ | | Gb |
| $CH_2$=$CHCH_2$ | H | Me | Gc |
| $CH_2$=$CHCH_2$ | H | Et | Gc |
| $CH_2$=$CHCH_2$ | Me | Me | Ga |

Table 1 (continued)

| $R^{11}$ | $R^{12}$ | $R^{13}$ | Gn |
|---|---|---|---|
| $CH_2=CHCH_2$ | Me | Et | Ga |
| $CH_2=CHCH_2$ | Me | Pr-n | Gb |
| $CH_2=CHCH_2$ | Me | Pr-i | Gb |
| $CH_2=CHCH_2$ | Me | Bu-n | Gb |
| $CH_2=CHCH_2$ | Me | Ph | Ga |
| $CH_2=CHCH_2$ | Me | $Ph-CH_2$ | Ga |
| $CH_2=CHCH_2$ | Et | Et | Gb |
| $CH_2=CHCH_2$ | Pr-n | Pr-n | Gb |
| $CH_2=CHCH_2$ | $-(CH_2)_4-$ | | Ga |
| $CH_2=CHCH_2$ | $-(CH_2)_5-$ | | Gb |
| $HC\equiv CCH_2$ | H | Me | Gc |
| $HC\equiv CCH_2$ | H | Et | Gc |
| $HC\equiv CCH_2$ | Me | Me | Ga |
| $HC\equiv CCH_2$ | Me | Et | Ga |
| $HC\equiv CCH_2$ | Me | Pr-n | Gb |
| $HC\equiv CCH_2$ | Me | Pr-i | Gb |
| $HC\equiv CCH_2$ | Me | Bu-n | Gb |
| $HC\equiv CCH_2$ | Me | Ph | Ga |
| $HC\equiv CCH_2$ | Me | $Ph-CH_2$ | Ga |
| $HC\equiv CCH_2$ | Et | Et | Gb |
| $HC\equiv CCH_2$ | Pr-n | Pr-n | Gb |
| $HC\equiv CCH_2$ | $-(CH_2)_4-$ | | Ga |
| $HC\equiv CCH_2$ | $-(CH_2)_5-$ | | Gb |
| $MeOCH_2$ | H | Me | Gc |
| $MeOCH_2$ | H | Et | Gc |

Table 1 (continued)

| $R^{11}$ | $R^{12}$ | $R^{13}$ | Gn |
|---|---|---|---|
| $MeOCH_2$ | Me | Me | Ga |
| $MeOCH_2$ | Me | Et | Ga |
| $MeOCH_2$ | Me | Pr-n | Gb |
| $MeOCH_2$ | Me | Pr-i | Gb |
| $MeOCH_2$ | Me | Bu-n | Gb |
| $MeOCH_2$ | Me | Ph | Ga |
| $MeOCH_2$ | Me | $Ph-CH_2$ | Ga |
| $MeOCH_2$ | Et | Et | Gb |
| $MeOCH_2$ | Pr-n | Pr-n | Gb |
| $MeOCH_2$ | $-(CH_2)_4-$ | | Ga |
| $MeOCH_2$ | $-(CH_2)_5-$ | | Gb |
| $Ph-CH_2$ | H | Me | Gc |

Table 1 (continued)

| $R^{11}$ | $R^{12}$ | $R^{13}$ | Gn |
|---|---|---|---|
| $Ph-CH_2$ | H | Et | Gc |
| $Ph-CH_2$ | Me | Me | Ga |
| $Ph-CH_2$ | Me | Et | Ga |
| $Ph-CH_2$ | Me | Pr-n | Gb |
| $Ph-CH_2$ | Me | Pr-i | Gb |
| $Ph-CH_2$ | Me | Bu-n | Gb |
| $Ph-CH_2$ | Me | Ph | Ga |
| $Ph-CH_2$ | Me | $Ph-CH_2$ | Ga |
| $Ph-CH_2$ | Et | Et | Gb |
| $Ph-CH_2$ | Pr-n | Pr-n | Gb |
| $Ph-CH_2$ | $-(CH_2)_4-$ | | Ga |
| $Ph-CH_2$ | $-(CH_2)_5-$ | | Gb |
| Pr-i | Me | Me | Ga |
| Pr-i | Me | Et | Ga |
| Pr-i | Me | Ph | Ga |
| Pr-i | Me | $Ph-CH_2$ | Ga |
| Pr-i | Et | Et | Gb |
| Pr-i | $-(CH_2)_4-$ | | Ga |
| Pr-i | $-(CH_2)_5-$ | | Gb |
| Bu-n | Me | Me | Ga |
| Bu-n | Me | Et | Ga |
| Bu-n | Me | Ph | Ga |
| Bu-n | Me | $Ph-CH_2$ | Ga |
| Bu-n | Et | Et | Gb |
| Bu-n | $-(CH_2)_4-$ | | Ga |

Table 1 (continued)

| $R^{11}$ | $R^{12}$ | $R^{13}$ | Gn |
|---|---|---|---|
| Bu-n | $-(CH_2)_5-$ | | Gb |
| Bu-s | Me | Me | Ga |
| Bu-s | Me | Et | Ga |
| Bu-s | Me | Ph | Ga |
| Bu-s | Me | $Ph-CH_2$ | Ga |
| Bu-s | Et | Et | Gb |
| Bu-s | $-(CH_2)_4-$ | | Ga |
| Bu-s | $-(CH_2)_5-$ | | Gb |
| Bu-i | Me | Me | Ga |
| Bu-i | Me | Et | Ga |
| Bu-i | Me | Ph | Ga |
| Bu-i | Me | $Ph-CH_2$ | Ga |
| Bu-i | Et | Et | Gb |
| Bu-i | $-(CH_2)_4-$ | | Ga |
| Bu-i | $-(CH_2)_5-$ | | Gb |
| Bu-t | Me | Me | Ga |
| Bu-t | Me | Et | Ga |
| Bu-t | Me | Ph | Ga |
| Bu-t | Me | $Ph-CH_2$ | Ga |
| Bu-t | Et | Et | Gb |
| Bu-t | $-(CH_2)_4-$ | | Ga |
| Bu-t | $-(CH_2)_5-$ | | Gb |
| Pen-n | Me | Me | Ga |
| Pen-n | Me | Et | Ga |
| Pen-n | Me | Ph | Ga |

Table 1 (continued)

| $R^{11}$ | $R^{12}$ | $R^{13}$ | Gn |
|---|---|---|---|
| Pen-n | Me | $Ph-CH_2$ | Ga |
| Pen-n | Et | Et | Gb |
| Pen-n | $-(CH_2)_4-$ | | Ga |
| Pen-n | $-(CH_2)_5-$ | | Gb |
| 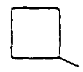 | Me | Me | Ga |
| 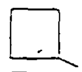 | Me | Et | Ga |
| 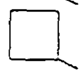 | Me | Ph | Ga |
| 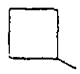 | Me | $Ph-CH_2$ | Ga |
| 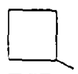 | Et | Et | Gb |
| 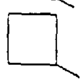 | $-(CH_2)_4-$ | | Ga |
| 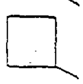 | $-(CH_2)_5-$ | | Gb |
| 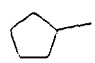 | Me | Me | Ga |
| 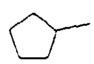 | Me | Et | Ga |
| 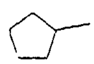 | Me | Ph | Ga |
| 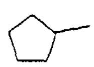 | Me | $Ph-CH_2$ | Ga |
| 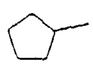 | Et | Et | Gb |
| 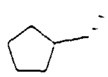 | $-(CH_2)_4-$ | | Ga |
| 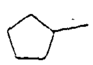 | $-(CH_2)_5-$ | | Gb |

Table 1 (continued)

| $R^{11}$ | $R^{12}$ | $R^{13}$ | Gn |
|---|---|---|---|
| 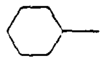 | Me | Me | Ga |
| 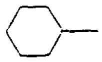 | Me | Et | Ga |
| 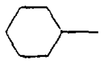 | Me | Ph | Ga |
| 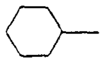 | Me | $Ph-CH_2$ | Ga |
| 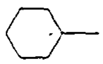 | Et | Et | Gb |
| 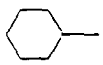 | $-(CH_2)_4-$ | | Ga |
| 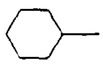 | $-(CH_2)_5-$ | | Gb |

Table 1 (continued)

| $R^{11}$ | $R^{12}$ | $R^{13}$ | Gn |
|---|---|---|---|
| $Me_2C=CH$ | Me | Me | Ga |
| $Me_2C=CH$ | Me | Et | Ga |
| $Me_2C=CH$ | Me | Ph | Ga |
| $Me_2C=CH$ | Me | $Ph-CH_2$ | Ga |
| $Me_2C=CH$ | Et | Et | Gb |
| $Me_2C=CH$ | $-(CH_2)_4-$ | | Ga |
| $Me_2C=CH$ | $-(CH_2)_5-$ | | Gb |
| $MeCH=CHCH_2$ | Me | Me | Ga |
| $MeCH=CHCH_2$ | Me | Et | Ga |
| $MeCH=CHCH_2$ | Me | Ph | Ga |
| $MeCH=CHCH_2$ | Me | $Ph-CH_2$ | Ga |
| $MeCH=CHCH_2$ | Et | Et | Gb |
| $MeCH=CHCH_2$ | $-(CH_2)_4-$ | | Ga |
| $MeCH=CHCH_2$ | $-(CH_2)_5-$ | | Gb |
| $CH_2=CHCH_2CH_2$ | Me | Me | Ga |

Table 1 (continued)

| $R^{11}$ | $R^{12}$ | $R^{13}$ | Gn |
|---|---|---|---|
| $CH_2=CHCH_2CH_2$ | Me | Et | Ga |
| $CH_2=CHCH_2CH_2$ | Me | Ph | Ga |
| $CH_2=CHCH_2CH_2$ | Me | $Ph-CH_2$ | Ga |
| $CH_2=CHCH_2CH_2$ | Et | Et | Gb |
| $CH_2=CHCH_2CH_2$ | $-(CH_2)_4-$ | | Ga |
| $CH_2=CHCH_2CH_2$ | $-(CH_2)_5-$ | | Gb |
| $CH_2=\overset{Me}{\overset{\mid}{C}}-CH_2$ | Me | Me | Ga |
| $CH_2=\overset{Me}{\overset{\mid}{C}}-CH_2$ | Me | Et | Ga |
| $CH_2=\overset{Me}{\overset{\mid}{C}}-CH_2$ | Me | Ph | Ga |
| $CH_2=\overset{Me}{\overset{\mid}{C}}-CH_2$ | Me | $Ph-CH_2$ | Ga |
| $CH_2=\overset{Me}{\overset{\mid}{C}}-CH_2$ | Et | Et | Gb |
| $CH_2=\overset{Me}{\overset{\mid}{C}}-CH_2$ | $-(CH_2)_4-$ | | Ga |
| $CH_2=\overset{Me}{\overset{\mid}{C}}-CH_2$ | $-(CH_2)_5-$ | | Gb |
| $CH_2=CH\overset{Me}{\overset{\mid}{C}}H$ | Me | Me | Ga |
| $CH_2=CH\overset{Me}{\overset{\mid}{C}}H$ | Me | Et | Ga |
| $CH_2=CH\overset{Me}{\overset{\mid}{C}}H$ | Me | Ph | Ga |

Table 1 (continued)

| $R^{11}$ | $R^{12}$ | $R^{13}$ | Gn |
|---|---|---|---|
| Me<br>$CH_2=CHCH$ | Me | $Ph-CH_2$ | Ga |
| Me<br>$CH_2=CHCH$ | Et | Et | Gb |
| Me<br>$CH_2=CHCH$ | $-(CH_2)_4-$ | | Ga |
| Me<br>$CH_2=CHCH$ | $-(CH_2)_5-$ | | Gb |
| $MeC\equiv CCH_2$ | Me | Me | Ga |
| $MeC\equiv CCH_2$ | Me | Et | Ga |
| $MeC\equiv CCH_2$ | Me | Ph | Ga |
| $MeC\equiv CCH_2$ | Me | $Ph-CH_2$ | Ga |
| $MeC\equiv CCH_2$ | Et | Et | Gb |
| $MeC\equiv CCH_2$ | $-(CH_2)_4-$ | | Ga |
| $MeC\equiv CCH_2$ | $-(CH_2)_5-$ | | Gb |
| Me<br>$HC\equiv CCH$ | Me | Me | Ga |
| Me<br>$HC\equiv CCH$ | Me | Et | Ga |
| Me<br>$HC\equiv CCH$ | Me | Ph | Ga |
| Me<br>$HC\equiv CCH$ | Me | $Ph-CH_2$ | Ga |
| Me<br>$HC\equiv CCH$ | Et | Et | Gb |

Table 1 (continued)

| $R^{11}$ | $R^{12}$ | $R^{13}$ | Gn |
|---|---|---|---|
| 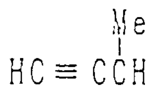 | $-(CH_2)_4-$ | | Ga |
| 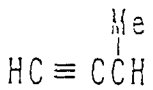 | $-(CH_2)_5-$ | | Gb |
| $MeOCH_2CH_2$ | Me | Me | Ga |
| $MeOCH_2CH_2$ | Me | Et | Ga |
| $MeOCH_2CH_2$ | Me | Ph | Ga |
| $MeOCH_2CH_2$ | Me | $Ph-CH_2$ | Ga |
| $MeOCH_2CH_2$ | Et | Et | Gb |
| $MeOCH_2CH_2$ | $-(CH_2)_4-$ | | Ga |
| $MeOCH_2CH_2$ | $-(CH_2)_5-$ | | Gb |
| $EtOCH_2CH_2$ | Me | Me | Ga |
| $EtOCH_2CH_2$ | Me | Et | Ga |
| $EtOCH_2CH_2$ | Me | Ph | Ga |
| $EtOCH_2CH_2$ | Me | $Ph-CH_2$ | Ga |
| $EtOCH_2CH_2$ | Et | Et | Gb |
| $EtOCH_2CH_2$ | $-(CH_2)_4-$ | | Ga |
| $EtOCH_2CH_2$ | $-(CH_2)_5-$ | | Gb |
| $MeOCMe_2$ | Me | Me | Ga |
| $MeOCMe_2$ | Me | Et | Ga |
| $MeOCMe_2$ | Me | Ph | Ga |
| $MeOCMe_2$ | Me | $Ph-CH_2$ | Ga |
| $MeOCMe_2$ | Et | Et | Gb |
| $MeOCMe_2$ | $-(CH_2)_4-$ | | Ga |
| $MeOCMe_2$ | $-(CH_2)_5-$ | | Gb |

Table 1 (continued)

| $R^{11}$ | $R^{12}$ | $R^{13}$ | Gn |
|---|---|---|---|
| $MeO\overset{Me}{\overset{\vert}{C}}HCH_2$ | Me | Me | Ga |
| $MeO\overset{Me}{\overset{\vert}{C}}HCH_2$ | Me | Et | Ga |
| $MeO\overset{Me}{\overset{\vert}{C}}HCH_2$ | Me | Ph | Ga |
| $MeO\overset{Me}{\overset{\vert}{C}}HCH_2$ | Me | $Ph-CH_2$ | Ga |
| $MeO\overset{Me}{\overset{\vert}{C}}HCH_2$ | Et | Et | Gb |
| $MeO\overset{Me}{\overset{\vert}{C}}HCH_2$ | $-(CH_2)_4-$ | | Ga |
| $MeO\overset{Me}{\overset{\vert}{C}}HCH_2$ | $-(CH_2)_5-$ | | Gb |
| $EtO-(CH_2)_4-$ | Me | Me | Ga |
| $EtO-(CH_2)_4-$ | Me | Et | Ga |
| $EtO-(CH_2)_4-$ | Me | Ph | Ga |
| $EtO-(CH_2)_4-$ | Me | $Ph-CH_2$ | Ga |
| $EtO-(CH_2)_4-$ | Et | Et | Gb |
| $EtO-(CH_2)_4-$ | $-(CH_2)_4-$ | | Ga |
| $EtO-(CH_2)_4-$ | $-(CH_2)_5-$ | | Gb |
| $MeSCH_2$ | Me | Me | Ga |
| $MeSCH_2$ | Me | Et | Ga |
| $MeSCH_2$ | Me | Ph | Ga |
| $MeSCH_2$ | Me | $Ph-CH_2$ | Ga |

Table 1 (continued)

| $R^{11}$ | $R^{12}$ | $R^{13}$ | Gn |
|---|---|---|---|
| $MeSCH_2$ | Et | Et | Gb |
| $MeSCH_2$ | $-(CH_2)_4-$ | | Ga |
| $MeSCH_2$ | $-(CH_2)_5-$ | | Gb |
| $MeSCH_2CH_2$ | Me | Me | Ga |
| $MeSCH_2CH_2$ | Me | Et | Ga |
| $MeSCH_2CH_2$ | Me | Ph | Ga |
| $MeSCH_2CH_2$ | Me | $Ph-CH_2$ | Ga |
| $MeSCH_2CH_2$ | Et | Et | Gb |
| $MeSCH_2CH_2$ | $-(CH_2)_4-$ | | Ga |
| $MeSCH_2CH_2$ | $-(CH_2)_5-$ | | Gb |
| $FCH_2CH_2$ | Me | Me | Ga |
| $FCH_2CH_2$ | Me | Et | Ga |
| $FCH_2CH_2$ | Me | Ph | Ga |
| $FCH_2CH_2$ | Me | $Ph-CH_2$ | Ga |
| $FCH_2CH_2$ | Et | Et | Gb |
| $FCH_2CH_2$ | $-(CH_2)_4-$ | | Ga |
| $FCH_2CH_2$ | $-(CH_2)_5-$ | | Gb |
| $C\ell CH_2CH_2$ | Me | Me | Ga |
| $C\ell CH_2CH_2$ | Me | Et | Ga |
| $C\ell CH_2CH_2$ | Me | Ph | Ga |
| $C\ell CH_2CH_2$ | Me | $Ph-CH_2$ | Ga |
| $C\ell CH_2CH_2$ | Et | Et | Gb |
| $C\ell CH_2CH_2$ | $-(CH_2)_4-$ | | Ga |
| $C\ell CH_2CH_2$ | $-(CH_2)_5-$ | | Gb |
| $BrCH_2CH_2$ | Me | Me | Ga |

Table 1 (continued)

| $R^{11}$ | $R^{12}$ | $R^{13}$ | Gn |
|---|---|---|---|
| $BrCH_2CH_2$ | Me | Et | Ga |
| $BrCH_2CH_2$ | Me | Ph | Ga |
| $BrCH_2CH_2$ | Me | $Ph-CH_2$ | Ga |
| $BrCH_2CH_2$ | Et | Et | Gb |
| $BrCH_2CH_2$ | $-(CH_2)_4-$ | | Ga |
| $BrCH_2CH_2$ | $-(CH_2)_5-$ | | Gb |
| $ClCH_2CH_2CH_2$ | Me | Me | Ga |
| $ClCH_2CH_2CH_2$ | Me | Et | Ga |
| $ClCH_2CH_2CH_2$ | Me | Ph | Ga |
| $ClCH_2CH_2CH_2$ | Me | $Ph-CH_2$ | Ga |
| $ClCH_2CH_2CH_2$ | Et | Et | Gb |
| $ClCH_2CH_2CH_2$ | $-(CH_2)_4-$ | | Ga |
| $ClCH_2CH_2CH_2$ | $-(CH_2)_5-$ | | Gb |
| $Cl-(CH_2)_4-$ | Me | Me | Ga |
| $Cl-(CH_2)_4-$ | Me | Et | Ga |
| $Cl-(CH_2)_4-$ | Me | Ph | Ga |
| $Cl-(CH_2)_4-$ | Me | $Ph-CH_2$ | Ga |
| $Cl-(CH_2)_4-$ | Et | Et | Gb |
| $Cl-(CH_2)_4-$ | $-(CH_2)_4-$ | | Ga |
| $Cl-(CH_2)_4-$ | $-(CH_2)_5-$ | | Gb |
| 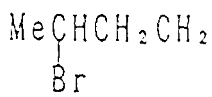 | Me | Me | Ga |
| 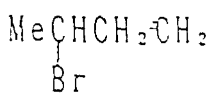 | Me | Et | Ga |
| 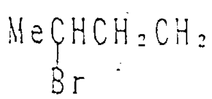 | Me | Ph | Ga |

Table 1 (continued)

| $R^{11}$ | $R^{12}$ | $R^{13}$ | Gn |
|---|---|---|---|
| $MeCHCH_2CH_2$<br>Br | Me | $Ph-CH_2$ | Ga |
| $MeCHCH_2CH_2$<br>Br | Et | Et | Gb |
| $MeCHCH_2CH_2$<br>Br | $-(CH_2)_4-$ | | Ga |
| $MeCHCH_2CH_2$<br>Br | $-(CH_2)_5-$ | | Gb |
| $NCCH_2$ | Me | Me | Ga |
| $NCCH_2$ | Me | Et | Ga |
| $NCCH_2$ | Me | Ph | Ga |
| $NCCH_2$ | Me | $Ph-CH_2$ | Ga |
| $NCCH_2$ | Et | Et | Gb |
| $NCCH_2$ | $-(CH_2)_4-$ | | Ga |
| $NCCH_2$ | $-(CH_2)_5-$ | | Gb |
| $NCCH_2CH_2$ | Me | Me | Ga |
| $NCCH_2CH_2$ | Me | Et | Ga |
| $NCCH_2CH_2$ | Me | Ph | Ga |
| $NCCH_2CH_2$ | Me | $Ph-CH_2$ | Ga |
| $NCCH_2CH_2$ | Et | Et | Gb |
| $NCCH_2CH_2$ | $-(CH_2)_4-$ | | Ga |
| $NCCH_2CH_2$ | $-(CH_2)_5-$ | | Gb |
| $NCCH_2CH_2CH_2$ | Me | Me | Ga |
| $NCCH_2CH_2CH_2$ | Me | Et | Ga |
| $NCCH_2CH_2CH_2$ | Me | Ph | Ga |

Table 1 (continued)

| $R^{11}$ | $R^{12}$ | $R^{13}$ | Gn |
|---|---|---|---|
| $NCCH_2CH_2CH_2$ | Me | $Ph-CH_2$ | Ga |
| $NCCH_2CH_2CH_2$ | Et | Et | Gb |
| $NCCH_2CH_2CH_2$ | $-(CH_2)_4-$ | | Ga |
| $NCCH_2CH_2CH_2$ | $-(CH_2)_5-$ | | Gb |
| $MeO_2CCH_2$ | Me | Me | Ga |
| $MeO_2CCH_2$ | Me | Et | Ga |
| $MeO_2CCH_2$ | Me | Ph | Ga |
| $MeO_2CCH_2$ | Me | $Ph-CH_2$ | Ga |
| $MeO_2CCH_2$ | Et | Et | Gb |
| $MeO_2CCH_2$ | $-(CH_2)_4-$ | | Ga |
| $MeO_2CCH_2$ | $-(CH_2)_5-$ | | Gb |
| $EtO_2CCH_2$ | Me | Me | Ga |
| $EtO_2CCH_2$ | Me | Et | Ga |
| $EtO_2CCH_2$ | Me | Ph | Ga |
| $EtO_2CCH_2$ | Me | $Ph-CH_2$ | Ga |
| $EtO_2CCH_2$ | Et | Et | Gb |
| $EtO_2CCH_2$ | $-(CH_2)_4-$ | | Ga |
| $EtO_2CCH_2$ | $-(CH_2)_5-$ | | Gb |
| $MeO_2C\overset{Me}{\overset{\vert}{C}}H$ | Me | Me | Ga |
| $MeO_2C\overset{Me}{\overset{\vert}{C}}H$ | Me | Et | Ga |
| $MeO_2C\overset{Me}{\overset{\vert}{C}}H$ | Me | Ph | Ga |
| $MeO_2C\overset{Me}{\overset{\vert}{C}}H$ | Me | $Ph-CH_2$ | Ga |

Table 1 (continued)

| $R^{11}$ | $R^{12}$ | $R^{13}$ | Gn |
|---|---|---|---|
| Me<br>\|<br>$MeO_2CCH$ | Et | Et | Gb |
| Me<br>\|<br>$MeO_2CCH$ | $-(CH_2)_4-$ | | Ga |
| Me<br>\|<br>$MeO_2CCH$ | $-(CH_2)_5-$ | | Gb |
| Me<br>\|<br>$EtO_2CCH$ | Me | Me | Ga |
| Me<br>\|<br>$EtO_2CCH$ | Me | Et | Ga |
| Me<br>\|<br>$EtO_2CCH$ | Me | Ph | Ga |
| Me<br>\|<br>$EtO_2CCH$ | Me | $Ph-CH_2$ | Ga |
| Me<br>\|<br>$EtO_2CCH$ | Et | Et | Gb |
| Me<br>\|<br>$EtO_2CCH$ | $-(CH_2)_4-$ | | Ga |
| Me<br>\|<br>$EtO_2CCH$ | $-(CH_2)_5-$ | | Gb |
| $MeO_2CCMe_2$ | Me | Me | Ga |
| $MeO_2CCMe_2$ | Me | Et | Ga |
| $MeO_2CCMe_2$ | Me | Ph | Ga |
| $MeO_2CCMe_2$ | Me | $Ph-CH_2$ | Ga |
| $MeO_2CCMe_2$ | Et | Et | Gb |

Table 1 (continued)

| $R^{11}$ | $R^{12}$ | $R^{13}$ | Gn |
|---|---|---|---|
| $MeO_2CCMe_2$ | $-(CH_2)_4-$ | | Ga |
| $MeO_2CCMe_2$ | $-(CH_2)_5-$ | | Gb |
| $EtO_2CCMe_2$ | Me | Me | Ga |
| $EtO_2CCMe_2$ | Me | Et | Ga |
| $EtO_2CCMe_2$ | Me | Ph | Ga |
| $EtO_2CCMe_2$ | Me | $Ph-CH_2$ | Ga |
| $EtO_2CCMe_2$ | Et | Et | Gb |
| $EtO_2CCMe_2$ | $-(CH_2)_4-$ | | Ga |
| $EtO_2CCMe_2$ | $-(CH_2)_5-$ | | Gb |
| $MeO_2CCH(Et)$ | Me | Me | Ga |
| $MeO_2CCH(Et)$ | Me | Et | Ga |
| $MeO_2CCH(Et)$ | Me | Ph | Ga |
| $MeO_2CCH(Et)$ | Me | $Ph-CH_2$ | Ga |
| $MeO_2CCH(Et)$ | Et | Et | Gb |
| $MeO_2CCH(Et)$ | $-(CH_2)_4-$ | | Ga |
| $MeO_2CCH(Et)$ | $-(CH_2)_5-$ | | Gb |
| $MeO_2CCH_2CH_2$ | Me | Me | Ga |
| $MeO_2CCH_2CH_2$ | Me | Et | Ga |

Table 1 (continued)

| $R^{11}$ | $R^{12}$ | $R^{13}$ | Gn |
|---|---|---|---|
| $MeO_2CCH_2CH_2$ | Me | Ph | Ga |
| $MeO_2CCH_2CH_2$ | Me | $Ph-CH_2$ | Ga |
| $MeO_2CCH_2CH_2$ | Et | Et | Gb |
| $MeO_2CCH_2CH_2$ | $-(CH_2)_4-$ | | Ga |
| $MeO_2CCH_2CH_2$ | $-(CH_2)_5-$ | | Gb |
| $EtO_2CCH_2CH_2$ | Me | Me | Ga |
| $EtO_2CCH_2CH_2$ | Me | Et | Ga |
| $EtO_2CCH_2CH_2$ | Me | Ph | Ga |
| $EtO_2CCH_2CH_2$ | Me | $Ph-CH_2$ | Ga |
| $EtO_2CCH_2CH_2$ | Et | Et | Gb |
| $EtO_2CCH_2CH_2$ | $-(CH_2)_4-$ | | Ga |
| $EtO_2CCH_2CH_2$ | $-(CH_2)_5-$ | | Gb |
| $MeO_2CCH_2CH_2CH_2$ | Me | Me | Ga |
| $MeO_2CCH_2CH_2CH_2$ | Me | Et | Ga |
| $MeO_2CCH_2CH_2CH_2$ | Me | Ph | Ga |
| $MeO_2CCH_2CH_2CH_2$ | Me | $Ph-CH_2$ | Ga |
| $MeO_2CCH_2CH_2CH_2$ | Et | Et | Gb |
| $MeO_2CCH_2CH_2CH_2$ | $-(CH_2)_4-$ | | Ga |
| $MeO_2CCH_2CH_2CH_2$ | $-(CH_2)_5-$ | | Gb |
| $EtO_2CCH_2CH_2CH_2$ | Me | Me | Ga |
| $EtO_2CCH_2CH_2CH_2$ | Me | Et | Ga |
| $EtO_2CCH_2CH_2CH_2$ | Me | Ph | Ga |
| $EtO_2CCH_2CH_2CH_2$ | Me | $Ph-CH_2$ | Ga |
| $EtO_2CCH_2CH_2CH_2$ | Et | Et | Gb |
| $EtO_2CCH_2CH_2CH_2$ | $-(CH_2)_4-$ | | Ga |

Table 1 (continued)

| $R^{11}$ | $R^{12}$ | $R^{13}$ | Gn |
|---|---|---|---|
| $EtO_2CCH_2CH_2CH_2$ | $-(CH_2)_5-$ | | Gb |
| $MeCOCH_2CMe_2$ | Me | Me | Ga |
| $MeCOCH_2CMe_2$ | Me | Et | Ga |
| $MeCOCH_2CMe_2$ | Me | Ph | Ga |
| $MeCOCH_2CMe_2$ | Me | $Ph-CH_2$ | Ga |
| $MeCOCH_2CMe_2$ | Et | Et | Gb |
| $MeCOCH_2CMe_2$ | $-(CH_2)_4-$ | | Ga |
| $MeCOCH_2CMe_2$ | $-(CH_2)_5-$ | | Gb |

Table 2

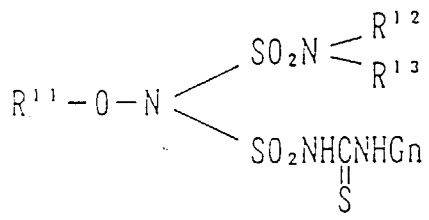

| $R^{11}$ | $R^{12}$ | $R^{13}$ | Gn |
|---|---|---|---|
| Me | Me | Me | Ga |
| Me | Me | Et | Gb |
| Me | $-(CH_2)_4-$ | | Gb |
| Me | $-(CH_2)_5-$ | | Gb |
| Et | Me | Me | Ga |
| Et | Me | Et | Gb |
| Et | $-(CH_2)_4-$ | | Gb |
| Et | $-(CH_2)_5-$ | | Gb |
| Pr-n | Me | Me | Ga |
| Pr-n | Me | Et | Gb |
| Pr-n | $-(CH_2)_4-$ | | Gb |
| Pr-n | $-(CH_2)_5-$ | | Gb |
| Pr-i | Me | Me | Ga |
| Pr-i | Me | Et | Gb |
| Pr-i | $-(CH_2)_4-$ | | Gb |
| Pr-i | $-(CH_2)_5-$ | | Gb |
| Pr-c | Me | Me | Ga |
| Pr-c | Me | Et | Gb |
| Pr-c | $-(CH_2)_4-$ | | Gb |
| Pr-c | $-(CH_2)_5-$ | | Gb |

Table 2 (continued)

| $R^{11}$ | $R^{12}$ | $R^{13}$ | Gn |
|---|---|---|---|
| $CH_2=CHCH_2$ | Me | Me | Ga |
| $CH_2=CHCH_2$ | Me | Et | Gb |
| $CH_2=CHCH_2$ | $-(CH_2)_4-$ | | Gb |
| $CH_2=CHCH_2$ | $-(CH_2)_5-$ | | Gb |
| $HC\equiv CCH_2$ | Me | Me | Ga |
| $HC\equiv CCH_2$ | Me | Et | Gb |
| $HC\equiv CCH_2$ | $-(CH_2)_4-$ | | Gb |
| $HC\equiv CCH_2$ | $-(CH_2)_5-$ | | Gb |
| Ph | Me | Me | Ga |
| Ph | Me | Et | Gb |
| Ph | $-(CH_2)_4-$ | | Gb |
| Ph | $-(CH_2)_5-$ | | Gb |
| Ph-$CH_2$ | Me | Me | Ga |
| Ph-$CH_2$ | Me | Et | Gb |
| Ph-$CH_2$ | $-(CH_2)_4-$ | | Gb |
| Ph-$CH_2$ | $-(CH_2)_5-$ | | Gb |

The above ingredients were homogeneously mixed and pulverized, and after an addition of a small amount of water, the mixture was stirred, mixed and granulated by an extrusion-type granulating machine, followed by drying to obtain a granule.

Further, the compound of the present invention may be combined with other herbicides, various insecticides, fungicides and synergism agents at the time of the preparation of the formulations or at the time of the application, as the case requires.

As such other herbicides, compound disclosed in Farm Chemicals Handbook (1989) may, for example, be mentioned.

For example, the compound of the present invention may be mixed with a herbicide such as alachlor, acetochlor, metolachlor, primisulfuron, nicosulfuron, atrazine, cyanazine, EPTC, 2,4-D, butylate, dicamba, bromoxynil, tridiphane, metsulfuron-methyl, thifensulfuron-methyl, triasulfuron, isoproturon, chlorotoluron, diflufenican, methabenzthiazuron, diclofop-methyl, difenzoquat, fenoxaprop-ethyl, bentazone, trifluralin, pendimethalin and N-[(4,6-dimethoxypyrimidin-2-yl)aminocarbonyl]-3-chloro-4-methoxycarbonyl-1-methylpyrazole-5-sulfonamide as disclosed in U.S. Patent 4,668,277, or with a herbicide safener (antidote) such as 1,8-naphthoic anhydride cr N,N-diallyl-2,2-dichloroacetamide.

The compound of the present invention can be applied to control various weeds not only in the agricultural and horticultural fields such as upland fields, paddy fields or orchards, but also in non-agricultural fields such as play grounds, non-used vacant fields or railway sides.

The dose varies depending upon the application site, the season for application, the manner of application, the type of weeds to be controlled, the type of crop plants, etc. However, the dose is usually within a range of from 0.005 to 10 kg per hectare as the amount of the active ingredient.

Now, the herbicidal activities of the compounds of the present invention will be described in detail with reference to the following Test Examples. The Compound Nos. referred to in the Test Examples correspond to the Compound Nos. given above.

TEST EXAMPLE 1: Test on the herbicidal effects in soil treatment

A plastic box having a length of 15 cm, a width of 22 cm and a depth of 6 cm was filled with a sterilized diluvial soil, and seeds of Echinochloa crus-galli, Digitaria adscendens, Cyperus microiria, Solanum nigrum, Galinsoga ciliata, Rorippa indica, Oryza sativa, Zea mays, Triticum aestivum, Glycine max and Gossypium herbaceum were sown, and the soil was covered thereon in the thickness of about 1.5 cm, and then a herbicide solution was applied onto the surface of the soil uniformly so that the active ingredient was distributed at a predetermined concentration. The herbicide solution was prepared by diluting the wettable powder as described in the foregoing Formulation Examples with water and applied onto the entire soil surface by means of a small spray. Four weeks after the application of the herbicidal solution, the herbicidal effects against each weed and the phytotoxicities against each crop plant were determined on the basis of the following standard ratings. The results are shown in Table 3. (Compound Nos. correspond to Compound Nos. in the Examples.) Some of the compounds of the present invention exhibit selectivity for certain crop plants.

Standard ratings:

5: Growth control rate of more than 90% (almost completely withered)
4: Growth control rate of from 70 to 90%
3: Growth control rate of from 40 to 70%
2: Growth control rate of from 20 to 40%
1: Growth control rate of from 5 to 20%
0: Growth control rate of less than 5% (almost non-effective)

The above growth control rates were calculated by the following equation:

$$\text{Growth control rate (\%)} = (1 - \frac{T}{N}) \times 100$$

where

T: Weight of the weed grown above the soil surface of the treated area
N: Weight of the weed grown above the soil surface of the non-treated area

TEST EXAMPLE 2: Test on the herbicidal effects in foliage treatment

A plastic box having a length of 15 cm, a width of 22 cm and a depth of 6 cm was filled with a sterilized diluvial soil, and seeds of Echinochloa crus-galli, Digitaria adscendens, Cyperus microiria, Solanum nigrum, Galinsoga ciliata, Rorippa indica, Ozyza sativa, Zea mays, Triticum aestivum, Glycine max, Gossypium herbaceum and Beta vulgaris were spot-wisely sown, and the soil was covered thereon in a thickness of about 1.5 cm. When the various weeds and crop plants grew to the 2 or 3 leaf stage, a herbicidal solution was uniformly sprayed on the foliages so that the active ingredient was applied in a predetermined concentration.

The herbicidal solution was prepared by diluting the wettable powder as described in the above Formulation Examples with water and applied onto the entire surface of the foliages of the weeds and the crop plants by a small spray. Four weeks after the application of the herbicide solution, the herbicidal effects against each weed and the phytotoxicities against each crop plant were determined on the basis of the standard ratings described in Test Example 1. The results are shown in Table 4. (Compound Nos. correspond to Compound Nos. in the Examples.)

In Tables 3 and 4, the following abbreviations are used.

Dose: Dose of active ingredient (kg/ha)
EC: Echinochloa crus-galli (barnyardgrass)
DI: Digitaria adscendens (large crabgrass)
CY: Cyperus microiria (annual sedge)
SO: Solanum nigrum (black nightshade)
GA: Galinsoga ciliate (hairy galinsoga)
RO: Rorippa indica (fieldcress)
OR: Oryza sativa (rice)
ZE: Zea mays (corn)
IF: Triticum aestivum (wheat)
GL: Glycine max (soybean)

GO: Gossypium herbaceum (cotton)
BE: Beta vulgaris (sugar beet)

Table 3

| Comp. No. | Dose kg/ha | EC | DI | CY | SO | GA | RO | OR | ZE | TR | GL | GO |
|---|---|---|---|---|---|---|---|---|---|---|---|---|
| 1 | 0.04 | 2 | 1 | 5 | 4 | 5 | 5 | 3 | 0 | 0 | 4 | 2 |
| | 0.08 | 3 | 2 | 5 | 5 | 5 | 5 | 4 | 1 | 0 | 5 | 3 |
| | 0.16 | 4 | 3 | 5 | 5 | 5 | 5 | 5 | 2 | 1 | 5 | 4 |
| 2 | 0.04 | 2 | 1 | 2 | 2 | 5 | 5 | 3 | 0 | 0 | 4 | 2 |
| | 0.08 | 3 | 2 | 3 | 4 | 5 | 5 | 4 | 1 | 0 | 5 | 3 |
| | 0.16 | 4 | 3 | 4 | 5 | 5 | 5 | 5 | 2 | 1 | 5 | 3 |
| 3 | 0.04 | 1 | 0 | 5 | 3 | 5 | 5 | 1 | 0 | 0 | 1 | 1 |
| | 0.08 | 2 | 1 | 5 | 4 | 5 | 5 | 2 | 0 | 0 | 2 | 2 |
| | 0.16 | 3 | 2 | 5 | 5 | 5 | 5 | 3 | 1 | 0 | 3 | 3 |
| 4 | 0.04 | 1 | 0 | 2 | 2 | 5 | 5 | 2 | 0 | 0 | 2 | 1 |
| | 0.08 | 2 | 1 | 4 | 4 | 5 | 5 | 3 | 0 | 0 | 3 | 2 |
| | 0.16 | 3 | 2 | 5 | 5 | 5 | 5 | 4 | 1 | 0 | 4 | 3 |
| 5 | 0.63 | 2 | 1 | 5 | 4 | 5 | 5 | 1 | 0 | 0 | 4 | 3 |
| 6 | 0.63 | 2 | 1 | 5 | 2 | 5 | 5 | 3 | 0 | 0 | 4 | 2 |

Table 3 (continued)

| Comp. No. | Dose kg/ha | EC | DI | CY | SO | GA | RO | OR | ZE | TR | GL | GO |
|---|---|---|---|---|---|---|---|---|---|---|---|---|
| 11 | 0.63 | 1 | 2 | 3 | 3 | 5 | 5 | 0 | 0 | 0 | 1 | 0 |
| | 0.16 | 1 | 1 | 2 | 4 | 5 | 5 | 4 | 1 | 1 | 4 | 1 |
| 13 | 0.32 | 2 | 2 | 3 | 5 | 5 | 5 | 5 | 2 | 2 | 5 | 2 |
| | 0.63 | 4 | 3 | 4 | 5 | 5 | 5 | 5 | 4 | 4 | 5 | 3 |
| 14 | 0.63 | 1 | 3 | 5 | 5 | 5 | 5 | 3 | 0 | 1 | 4 | 2 |
| 15 | 0.63 | 3 | 2 | 5 | 5 | 5 | 5 | 4 | 0 | 0 | 2 | 3 |

Table 3 (continued)

| Comp. No. | Dose kg/ha | EC | DI | CY | SO | GA | RO | OR | ZE | TR | GL | GO |
|---|---|---|---|---|---|---|---|---|---|---|---|---|
| 16 | 0.63 | 1 | 2 | 4 | 4 | 5 | 5 | 3 | 0 | 1 | 3 | 1 |
| | 0.16 | 2 | 1 | 5 | 5 | 5 | 5 | 1 | 0 | 0 | 2 | 0 |
| 18 | 0.32 | 3 | 2 | 5 | 5 | 5 | 5 | 2 | 0 | 0 | 3 | 1 |
| | 0.63 | 4 | 3 | 5 | 5 | 5 | 5 | 4 | 1 | 1 | 4 | 2 |

Table 4

| Comp. No. | Dose kg/ha | EC | DI | CY | SO | GA | RO | OR | ZE | TR | GL | GO | BE |
|---|---|---|---|---|---|---|---|---|---|---|---|---|---|
| 1 | 0.04 | 2 | 1 | 4 | 5 | 5 | 5 | 1 | 0 | 0 | 4 | 4 | 5 |
| | 0.08 | 3 | 2 | 5 | 5 | 5 | 5 | 2 | 0 | 0 | 5 | 5 | 5 |
| | 0.16 | 4 | 3 | 5 | 5 | 5 | 5 | 3 | 1 | 0 | 5 | 5 | 5 |
| 2 | 0.04 | 2 | 1 | 2 | 4 | 5 | 5 | 2 | 0 | 0 | 4 | 4 | 4 |
| | 0.08 | 3 | 2 | 3 | 5 | 5 | 5 | 3 | 1 | 0 | 5 | 5 | 5 |
| | 0.16 | 4 | 3 | 4 | 5 | 5 | 5 | 4 | 3 | 1 | 5 | 5 | 5 |
| 3 | 0.04 | 1 | 0 | 4 | 4 | 5 | 5 | 0 | 0 | 0 | 4 | 4 | 5 |
| | 0.08 | 2 | 1 | 5 | 5 | 5 | 5 | 1 | 0 | 0 | 5 | 5 | 5 |
| | 0.16 | 3 | 2 | 5 | 5 | 5 | 5 | 2 | 1 | 0 | 5 | 5 | 5 |
| 4 | 0.04 | 1 | 0 | 1 | 4 | 5 | 5 | 0 | 0 | 0 | 3 | 4 | 5 |
| | 0.08 | 2 | 1 | 2 | 5 | 5 | 5 | 0 | 0 | 0 | 4 | 5 | 5 |
| | 0.16 | 3 | 2 | 3 | 5 | 5 | 5 | 1 | 1 | 0 | 5 | 5 | 5 |
| 5 | 0.63 | 3 | 2 | 5 | 5 | 5 | 5 | 1 | 1 | 0 | 4 | 5 | 5 |
| 6 | 0.63 | 2 | 1 | 5 | 2 | 5 | 5 | 1 | 0 | 0 | 4 | 5 | 5 |

Table 4 (continued)

| Comp. No. | Dose kg/ha | EC | DI | CY | SO | GA | RO | OR | ZE | TR | GL | GO | BE |
|---|---|---|---|---|---|---|---|---|---|---|---|---|---|
| 11 | 0.63 | 1 | 1 | 4 | 4 | 5 | 5 | 0 | 1 | 0 | 3 | 4 | 4 |
| 13 | 0.16 | 2 | 1 | 1 | 5 | 5 | 5 | 3 | 0 | 0 | 4 | 2 | 5 |
| | 0.32 | 3 | 2 | 2 | 5 | 5 | 5 | 4 | 1 | 1 | 5 | 3 | 5 |
| | 0.63 | 4 | 3 | 2 | 5 | 5 | 5 | 5 | 3 | 3 | 5 | 3 | 5 |
| 14 | 0.63 | 3 | 3 | 2 | 5 | 5 | 5 | 2 | 0 | 0 | 4 | 2 | 5 |
| 15 | 0.63 | 2 | 1 | 4 | 4 | 5 | 5 | 2 | 3 | 0 | 3 | 2 | 5 |
| 16 | 0.63 | 2 | 1 | 2 | 4 | 5 | 5 | 0 | 1 | 0 | 4 | 2 | 4 |
| 18 | 0.63 | 2 | 3 | 3 | 4 | 5 | 5 | 2 | 3 | 2 | 4 | 2 | 4 |

Next, the present inventors have conducted a research with an aim to increase the herbicidal effects of the compound of the present invention. As a result, it has been found that when one of alachlor, acetochlor, metolachlor, primisulfuron and nicosulfuron is incorporated to the compound of the present invention, not only the herbicidal effects of the respective compounds appear as their sum, but a synergistic effect is obtainable without presenting phytotoxicity to crop plants.

The synergistic herbicidal effects of a mixture of herbicides will be explained as follows.

Namely, the individual active compounds may show drawbacks of their own in the herbicidal activities in many cases. In such a case, when two active compounds are combined, if the herbicidal activities thereby obtained are larger than the simple sum of the respective activities of the two compounds (i.e. the expected activities), this is regarded as a synergistic effect. The expected activities by a specific combination of two herbicides are calculated as follows. (See Colby S.R. Calculation of the synergistic and antagonistic activities by combinations of herbicides "Weed", vol 15, p. 20-22 (1967)).

$$E = \alpha + \beta - \frac{\alpha \cdot \beta}{100}$$

where $\alpha$ is the control rate when herbicide A was applied at a dose of a kg/ha, $\beta$ is the control rate when herbicide B was applied at a dose of b kg/ha, and E is the expected control rate when a kg/ha of herbicide A and b kg/ha of herbicide B are applied.

Namely, if the actual control rate is larger than the control rate calculated by the above formula, the activities by the combination are regarded as exhibiting a synergistic effect.

Now, this will be specifically explained with reference to Examples. However, the compounds, the proportions in the formulations, and the types of formulation according to the present invention should not be regarded as limited to such specific Examples. In these Examples, "parts" means "parts by weight".

FORMULATION EXAMPLE 5: Wettable powder

| | |
|---|---|
| Compound No. 3 of the present invention | 10 parts |
| Primisulfuron | 10 parts |
| Zeeklite PFP (tradename for a kaolin-type clay, manufactured by Zeeklite Industries, Co., Ltd.) | 72 parts |
| Sorpol 5039 (tradename for an alkylether sulfate, manufactured by Toho Chemical Industry Co., Ltd.) | 2 parts |
| Soybean oil | 2 parts |
| Carplex #80 (tradename for fine silica powder, manufactured by Sionogi Pharmaceutical Co., Ltd. | 4 parts |

The above ingredients are homogeneously pulverized and mixed to form a wettable powder.

FORMULATION EXAMPLE 6: Wettable powder

| | |
|---|---|
| Compound No. 3 of the present invention | 10 parts |
| Nicosulfuron | 10 parts |
| Zeeklite PFP (tradename for a kaolin-type clay, manufactured by Zeeklite Industries, Co., Ltd.) | 72 parts |
| Sorpol 5039 (tradename for an alkylether sulfate, manufactured by Toho Chemical Industry Co., Ltd.) | 2 parts |
| Soybean oil | 2 parts |
| Carplex #80 (tradename for fine silica powder, manufactured by Sionogi Pharmaceutical Co., Ltd. | 4 parts |

The above ingredients are homogeneously pulverized and mixed to form a wettable powder.

FORMULATION EXAMPLE 7: Suspension concentrate

| | |
|---|---|
| Compound No. 3 of the present invention | 2 parts |
| Alachlor | 38 parts |
| Sorbon T-80 (tradename for polyoxyethylene sorbitan monooleate, manufactured by Toho Chemical Industry Co., Ltd.) | 5 parts |
| Vigum (tradename for magnesium·aluminium·silicate, manufactured by Vanderbil Co.) | 5 parts |
| Water | 50 parts |

The above ingredients were uniformly mixed and pulverized by a sand mill to obtain a suspension concentrate having a particle size of at most 5 µm.

FORMULATION EXAMPLE 8 : Suspension concentrate

| | |
|---|---|
| Compound No. 3 of the present invention | 2 parts |
| Acetochlor | 38 parts |
| Sorbon T-80 (tradename for polyoxyethylene sorbitan monooleate, manufactured by Toho Chemical Industry Co., Ltd.) | 5 parts |
| Vigum (tradename for magnesium·aluminium·silicate, manufactured by Vanderbil Co.) | 5 parts |
| Water | 50 parts |

The above ingredients were uniformly mixed and pulverized by a sand mill to obtain a suspension concentrate having a particle size of at most 5 µm.

FORMULATION EXAMPLE 9 : Granule

| | |
|---|---|
| Compound No. 3 of the present invention | 5 parts |
| Metolachlor | 75 parts |
| Aron A (tradename for a polycarboxylate, manufactured by Toagosei Chemical Industry Co., Ltd.) | 7 parts |
| Sanekisu C (tradename for lignin sulfonate, manufactured by Sanyo-Kokusaku Pulp Co., Ltd. | 10 parts |
| Newlex powder (tradename for DBS-Na: manufactured by Nippon Oil and Fats Co., Ltd.) | 2 parts |
| Zeeklite PFP (tradename for a kaolin-type clay, manufactured by Zeeklite Industries, Co., Ltd.) | 1 part |

To the above ingredients, a suitable amount of water was added, and the mixture was pulverized and mixed to obtain a slurry, which was granulated to a granule while removing the moisture by a spray dryer.

FORMULATION EXAMPLE 10: Granule

| | |
|---|---|
| Compound No. 3 of the present invention | 5 parts |
| Alachlor | 75 parts |
| Aron A (tradename for a polycarboxylate, manufactured by Toagosei Chemical Industry Co., Ltd.) | 7 parts |
| Sanekisu C (tradename for lignin sulfonate, manufactured by Sanyo-Kokusaku Pulp Co., Ltd. | 10 parts |
| Newlex powder (tradename for DBS-Na: manufactured by Nippon Oil and Fats Co., Ltd.) | 2 parts |
| Zeeklite PFP (tradename for a kaolin-type clay, manufactured by Zeeklite Industries, Co., Ltd.) | 1 part |

To the above ingredients, a suitable amount of water was added, and the mixture was pulverized and mixed to obtain a slurry, which was granulated to a granule while removing the moisture by a spray dryer.

TEST EXAMPLE 3: Test on the herbicidal effects in soil treatment

A plastic box having a length of 30 cm, a width of 30 cm and a depth of 10 cm was filled with a sterilized upland soil, and seeds of Zea mays (corn), Setaria viridis (green foxtail), Abutilon theophrasti (velvet leaf) and Xanthium strumarium (cocklebur) were spot-wisely sown, and the soil was covered thereon in the thickness of about 0.5 cm. The herbicide solution was applied on the same day as the seeding in a sprayed water amount of 5 ℓ/a. The herbicidal solution applied was prepared by diluting the formulation as described in the foregoing Formulation Examples with water and applied by means of a small spray. The weight of each weed grown above the soil surface was measured upon expiration of three weeks from the application, and the growth control rate (%) was calculated by the following equation.

$$\text{Growth control rate (\%)} = (1 - \frac{T}{N}) \times 100$$

where

T: weight of the weed grown above the soil surface of the treated area;
N: weight of the weed grown above the soil surface of the non-treated area.

The results of the test for the herbicidal effects by the individual herbicides are shown in Table 5 . The results of the test for the herbicidal effects of the mixture of the herbicides are shown in Table 6 . (Compound Nos. correspond to Compound Nos. described in the Examples.)

TEST EXAMPLE 4: Test on the herbicidal effects in the treatment during the growing stage

A plastic box having a length of 30 cm, a width of 30 cm and a depth of 10 cm was filled with a sterilized upland soil, and seeds of Zea mays (corn), Setaria viridis (green foxtail), Abutilon theophrasti (velvet leaf) and Xanthium strumarium (cocklebur) were spot-wisely sown, and the soil was covered thereon in a thickness of 0.5 cm. On the tenth day after the seeding, the growing stage spraying of a herbicidal solution was conducted at a sprayed water amount of 5 ℓ/a. The herbicidal solution for spraying was prepared by diluting the formulations prepared in accordance with the preceding Formulation Examples with water and sprayed by means of a small spray. The weight of each weed grown above the soil surface was measured upon expiraticn of two weeks after the application, and the growth control rate (%) was obtained in the same manner as -in Test Example 3.

The results of the test for the herbicidal effects of the individual herbicides are shown in Table 7.

The results of the test for the herbicidal effects of the mixture of the herbicides are shown in Table 8 . (Compound Nos. correspond to Compound Nos. described in Examples.)

In Tables 5 to 8 , the following abbreviations are used.

Dose: Dose of active ingredient (g/a)
SE: Setaria viridis (green foxtail)
AB: Abutilon theophrasti (velvet leaf)
XA: Xanthium strumarium (cocklebur)
ZE: Zea mays (corn)
mv: Measured value
ev: Expected value

Table 5

| Compound | Dose g/a | SE | AB | XA | ZE |
|---|---|---|---|---|---|
| No. 3 | 0.01 | 0 | 35 | 46 | 0 |
| | 0.03 | 0 | 48 | 68 | 0 |
| | 0.1 | 6 | 85 | 88 | 0 |
| Alachlor | 0.1 | 28 | 0 | 0 | 0 |
| | 0.3 | 53 | 0 | 0 | 0 |
| | 1.0 | 87 | 4 | 0 | 0 |
| Acetochlor | 0.1 | 36 | 0 | 0 | 0 |
| | 0.3 | 74 | 0 | 0 | 0 |
| | 1.0 | 92 | 8 | 0 | 0 |
| Metolachlor | 0.1 | 32 | 0 | 0 | 0 |
| | 0.3 | 70 | 0 | 0 | 0 |
| | 1.0 | 97 | 7 | 0 | 0 |

Table 6

| Mixture No. 3 + C | Dose g/a | SE | | AB | | XA | | ZE | |
|---|---|---|---|---|---|---|---|---|---|
| C | No. 3 + C | mv | ev | mv | ev | mv | ev | mv | ev |
| Alachlor | 0.01 + 0.1 | 39 | 28 | 46 | 35 | 50 | 46 | 0 | 0 |
| | 0.01 + 0.3 | 62 | 53 | 48 | 35 | 53 | 46 | 0 | 0 |
| | 0.01 + 1 | 98 | 87 | 52 | 38 | 54 | 46 | 0 | 0 |
| | 0.03 + 0.1 | 43 | 28 | 56 | 48 | 79 | 68 | 0 | 0 |
| | 0.03 + 0.3 | 66 | 53 | 60 | 48 | 80 | 68 | 0 | 0 |
| | 0.03 + 1 | 99 | 87 | 63 | 50 | 85 | 68 | 0 | 0 |
| | 0.1 + 0.1 | 46 | 32 | 95 | 85 | 98 | 88 | 0 | 0 |
| | 0.1 + 0.3 | 72 | 56 | 99 | 85 | 98 | 88 | 0 | 0 |
| | 0.1 + 1 | 100 | 88 | 100 | 86 | 100 | 88 | 0 | 0 |
| Acetochlor | 0.01 + 0.1 | 43 | 36 | 41 | 35 | 52 | 46 | 0 | 0 |
| | 0.01 + 0.3 | 82 | 74 | 43 | 35 | 56 | 46 | 0 | 0 |
| | 0.01 + 1 | 100 | 92 | 50 | 40 | 59 | 46 | 0 | 0 |
| | 0.03 + 0.1 | 50 | 36 | 61 | 48 | 72 | 68 | 0 | 0 |
| | 0.03 + 0.3 | 83 | 74 | 65 | 48 | 76 | 68 | 0 | 0 |
| | 0.03 + 1 | 100 | 92 | 70 | 52 | 80 | 68 | 0 | 0 |
| | 0.1 + 0.1 | 56 | 40 | 92 | 85 | 95 | 88 | 0 | 0 |
| | 0.1 + 0.3 | 88 | 76 | 95 | 85 | 98 | 88 | 0 | 0 |
| | 0.1 + 1 | 100 | 92 | 100 | 86 | 100 | 88 | 0 | 0 |
| Metolachlor | 0.01 + 0.1 | 43 | 32 | 44 | 35 | 52 | 46 | 0 | 0 |
| | 0.01 + 0.3 | 82 | 70 | 46 | 35 | 56 | 46 | 0 | 0 |
| | 0.01 + 1 | 100 | 97 | 51 | 40 | 60 | 46 | 0 | 0 |
| | 0.03 + 0.1 | 45 | 32 | 60 | 48 | 73 | 68 | 0 | 0 |
| | 0.03 + 0.3 | 84 | 70 | 60 | 48 | 74 | 68 | 0 | 0 |
| | 0.03 + 1 | 100 | 97 | 68 | 52 | 79 | 68 | 0 | 0 |
| | 0.1 + 0.1 | 46 | 36 | 99 | 85 | 94 | 88 | 0 | 0 |
| | 0.1 + 0.3 | 85 | 72 | 100 | 85 | 97 | 88 | 0 | 0 |
| | 0.1 + 1 | 100 | 97 | 100 | 86 | 100 | 88 | 0 | 0 |

(The expected value is the value calculated by the Colby's equation mentioned before. )

Table 7

| Compound | Dose g/a | SE | AB | XA | ZE |
|---|---|---|---|---|---|
| No. 3 | 0.01 | 0 | 42 | 58 | 0 |
| | 0.03 | 0 | 79 | 75 | 0 |
| | 0.1 | 12 | 96 | 97 | 0 |

Table 7 (continued)

| Compound | Dose g/a | SE | AB | XA | ZE |
|---|---|---|---|---|---|
| Primisulfuron | 0.01 | 10 | 32 | 46 | 0 |
| | 0.03 | 23 | 71 | 70 | 0 |
| | 0.1 | 58 | 89 | 91 | 0 |
| Nicosulfuron | 0.01 | 36 | 4 | 2 | 0 |
| | 0.03 | 72 | 12 | 20 | 0 |
| | 0.1 | 87 | 40 | 38 | 0 |

Table 8

| Mixture No. 3 + C | Dose g/a | SE | | AB | | XA | | ZE | |
|---|---|---|---|---|---|---|---|---|---|
| C | No. 3 + C | mv | ev | mv | ev | mv | ev | mv | ev |
| Primisulfuron | 0.01 + 0.01 | 28 | 10 | 77 | 61 | 86 | 77 | 0 | 0 |
| | 0.01 + 0.03 | 46 | 23 | 90 | 83 | 99 | 87 | 0 | 0 |
| | 0.01 + 0.1 | 75 | 58 | 100 | 94 | 100 | 96 | 0 | 0 |
| | 0.03 + 0.01 | 31 | 10 | 99 | 86 | 98 | 87 | 0 | 0 |
| | 0.03 + 0.03 | 49 | 23 | 100 | 94 | 100 | 93 | 0 | 0 |
| | 0.03 + 0.1 | 80 | 58 | 100 | 98 | 100 | 98 | 0 | 0 |
| | 0.1 + 0.01 | 36 | 21 | 100 | 97 | 100 | 98 | 0 | 0 |
| | 0.1 + 0.03 | 55 | 32 | 100 | 99 | 100 | 99 | 0 | 0 |
| | 0.1 + 0.1 | 84 | 63 | 100 | 100 | 100 | 100 | 0 | 0 |
| Nicosulfuron | 0.01 + 0.01 | 53 | 36 | 61 | 44 | 73 | 59 | 0 | 0 |
| | 0.01 + 0.03 | 89 | 72 | 68 | 49 | 80 | 66 | 0 | 0 |
| | 0.01 + 0.1 | 99 | 87 | 84 | 65 | 85 | 74 | 0 | 0 |
| | 0.03 + 0.01 | 54 | 36 | 95 | 80 | 90 | 76 | 0 | 0 |
| | 0.03 + 0.03 | 86 | 72 | 95 | 82 | 91 | 80 | 0 | 0 |
| | 0.03 + 0.1 | 99 | 87 | 99 | 87 | 98 | 85 | 0 | 0 |
| | 0.1 + 0.01 | 60 | 42 | 100 | 96 | 100 | 97 | 0 | 0 |
| | 0.1 + 0.03 | 89 | 75 | 100 | 96 | 100 | 98 | 0 | 0 |
| | 0.1 + 0.1 | 100 | 89 | 100 | 98 | 100 | 98 | 0 | 0 |

(The expected value is the value calculated by the Colby's equation mentioned before. )

## Claims

**1.** A sulfamidosulfonylurea derivative of the formula (1) or an agriculturally suitable salt thereof:

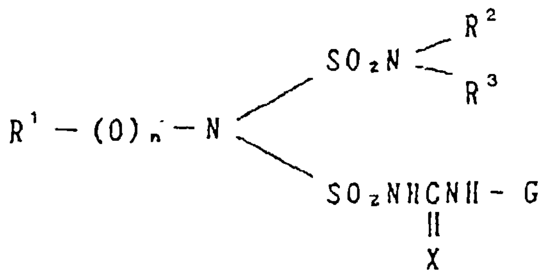

(I)

wherein $R^1$ is a hydrogen atom, a $C_1$-$C_6$ lower alkyl group, a $C_3$-$C_7$ cycloalkyl group, a $C_2$-$C_6$ lower alkenyl group, a $C_2$-$C_6$ lower alkynyl group, a $C_1$-$C_6$ lower alkyl group, substituted by a $C_1$-$C_6$ lower alkoxy group, a $C_1$-$C_6$ lower alkyl group substiuted by a $C_1$-$C_6$ lower mono- or polyhalogenoalkoxy group, a $C_1$-$C_6$ lower alkyl group substituted by a $C_1$-$C_6$ lower alkylthio group, a $C_1$-$C_6$ lower alkyl group substituted by a $C_1$-$C_6$ lower alkylsulfonyl group, a $C_1$-$C_6$ lower mono- or polyhalogenoalkyl group, a $C_1$-$C_6$ lower alkyl group substituted by a cyano group, a $C_1$-$C_6$ lower alkyl group substituted by a $C_1$-$C_6$ lower alkoxycarbonyl group, a $C_1$-$C_6$ lower alkyl group substituted by a $C_1$-$C_6$ lower alkylcarbonyl group, a benzyl group (provided that such a benzyl group may be substituted by a halogen atom, a trifluoromethyl group, a $C_1$-$C_6$ lower alkyl group, a $C_1$-$C_6$ lower alkoxy group or a $C_1$-$C_6$ lower alkoxycarbonyl group), $R^2$ is a hydrogen atom or a $C_1$-$C_6$ lower alkyl group, $R^3$ is a $C_1$-$C_6$ lower alkyl group, a phenyl group or a benzyl group (provided that such a phenyl group or a benzyl group may be substituted by a halogen atom, a trifluoromethyl group, a $C_1$-$C_6$ lower alkyl group, a $C_1$-$C_6$ lower alkoxy group, a $C_1$-$C_6$ lower alkoxycarbonyl group or a nitro group), or $R^2$ and $R^3$ form, together with the nitrogen atom to which they are bonded, a saturated 5-7 membered heterocyclic group;
n is 1;
X is an oxygen atom or a sulfur atom; and
G is

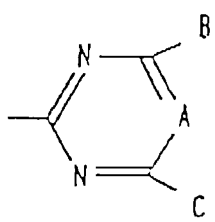

wherein A is a CH group or a nitrogen atom, and each of B and C independently is a $C_1$-$C_4$ lower alkyl group, a $C_1$-$C_4$ lower alkoxy group, a $C_1$-$C_4$ lower halogenoalkyl group, a $C_1$-$C_4$ lower halogenoalkoxy group, a halogen atom or a $C_1$-$C_4$ lower mono-alkylamino group.

2. The compound according to Claim 1, wherein $R^1$ is a $C_2H_5$ group, $R^2$ is a $CH_3$ group, $R^3$ is a $CH_3$ group, A is a CH-group, B is a $CH_3O$ group, and C is a $CH_3O$ group.

3. The compound according to Claim 1, wherein $R^1$ is a $CH_3$ group, $R^2$ is a $CH_3$ group, $R^3$ is a $CH_3$ group, A is a CH group, B is a $CH_3O$ group and C is a $CH_3O$ group.

4. A selective herbicide containing one or more compounds defined in one of claims 1 to 3 as active ingredients.

5. A herbicidal composition comprising one or more compounds defined in one of claims 1 to 3 and a member selected from the group consisiting of alachlor, acetochlor, metolachlor, primisulfuron and nicosulfuron, as active ingredients.

6. A sulfamidosulfonylisocyanate compound of the formula (II):

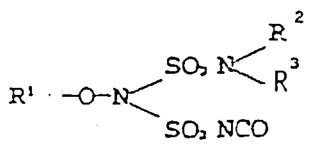

(II)

wherein $R^1$, $R^2$ and $R^3$ are defined as in Claim 1.

**7.** A sulfamidosulfonamide compound of the formula (IV):

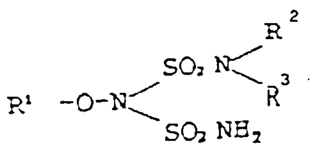

(IV)

wherein $R^1$, $R^2$ and $R^3$ are defined as in claim 1.

**8.** A sulfamidosulfonylcarbamate compound of the formula (V):

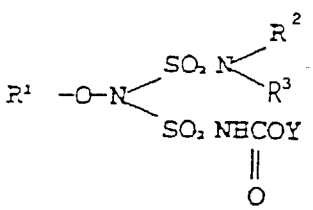

(V)

wherein R1, $R^2$ and $R^3$ are defined as in Claim 1 and Y is a $C_1$-$C_6$ alkyl group or phenyl group.

**9.** An N-(t-butylsulfamoyl)sulfamide compound of the formula (XI):

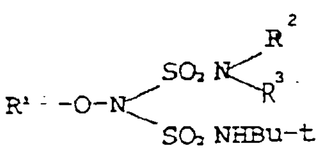

(XI)

wherein R1, $R^2$ and $R^3$ are defined as in Claim 1.

**10.** An alkoxysulfamide compound of the formula (VI):

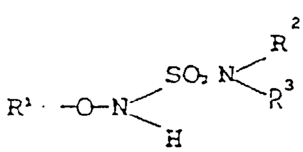

(VI)

wherein $R^1$, $R^2$ and $R^3$ are defined as in Claim 1.

**11.** A process for producing a sulfamidosulfonylurea compound of the formula (I):

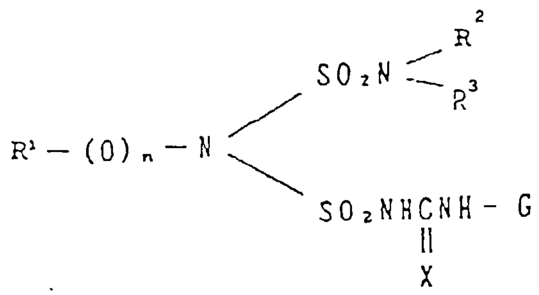

(I)

wherein $R^1$, $R^2$, $R^3$ and n are defined as in Claim 1, which comprises:

(a) reacting a sulfamidosulfonylisocyanate compound of the formula (II):

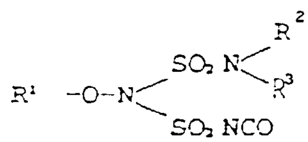

(II)

wherein $R^1$, $R^2$ and $R^3$ are as defined above, with a pyrimidine compound of the formula (III):

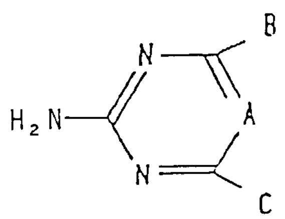

(III)

wherein A, B and C are as defined above, to obtain a compound of the formula (I);

(b) reacting a sulfamidosulfonamide compound of the formula (IV):

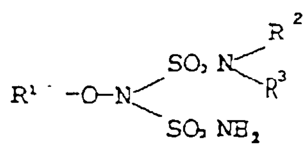

(IV)

wherein $R^1$, $R^2$ and $R^3$ are as defined above, with a carbamate compound of the formula (VIII):

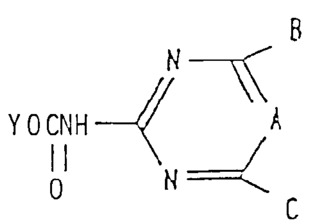

(VIII)

wherein A, B and C are as defined above, and Y is a $C_1$-$C_6$ alkyl group or phenyl group, to obtain a compound of the formula (I);

(c) reacting a sulfamidosulfonylcarbamate compound of the formula (V):

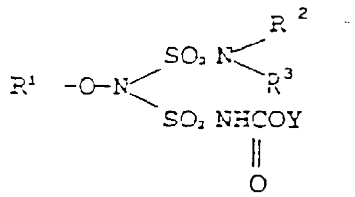

(V)

wherein $R^1$, $R^2$ and $R^3$ are as defined above, and Y is a $C_1$-$C_6$ alkyl group or phenyl group, with a pyrimidine copound of the formula (III):

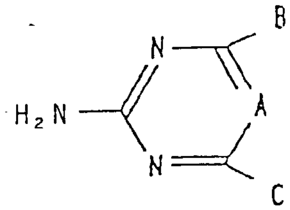

(III)

wherein A, B and C are as defined above, to obtain a compound of the formula (I);
(d) reacting a pyrimidine compound of the formula (III):

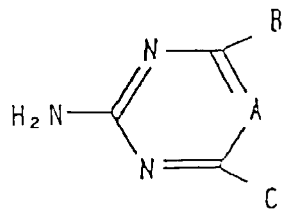

(III)

wherein A, B and C are as defined above, with chlorosulfonylisocyanate, and then reacted with a sulfamide compound of the formula (VI):

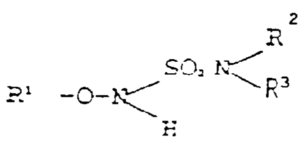

(VI)

wherein $R^1$, $R^2$ and $R^3$ are as defined above, to obtain a compound of the formula (I).

## Patentansprüche

1. Ein Sulfamidosulfonylharnstoffderivat der Formel (1) oder ein landwirtschaftlich geeignetes Salz davon:

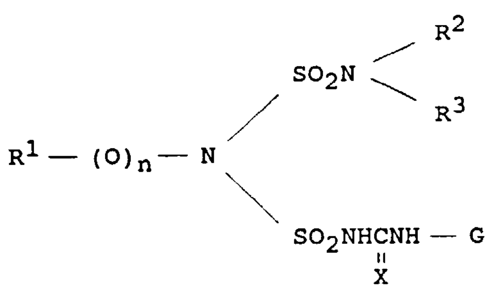

(I)

worin $R^1$ ein Wasserstoffatom, eine niedere $C_1$-$C_6$-Alkylgruppe, eine $C_3$-$C_7$-Cycloalkylgrupe, eine niedere $C_2$-$C_6$-Alkenylgruppe, eine niedere $C_2$-$C_6$-Alkinylgruppe, eine durch eine niedere $C_1$-$C_6$-Alkoxygruppe substituierte niedere $C_1$-$C_6$-Alkylgruppe, eine durch eine niedere $C_1$-$C_6$-Mono- oder Polyhalogenalkoxygruppe substituierte niedere $C_1$-$C_6$-Alkylgruppe, eine durch eine niedere $C_1$-$C_6$-Alkylthiogruppe substituierte niedere $C_1$-$C_6$-Alkylgruppe, eine durch eine niedere $C_1$-$C_6$-Alkylsulfonylgruppe substituierte niedere $C_1$-$C_6$-Alkylgruppe, eine niedere $C_1$-$C_6$-Mono- oder Polyhalogenalkylgruppe, eine durch eine Cyanogruppe substituierte niedere $C_1$-$C_6$-Alkylgruppe, eine durch eine niedere $C_1$-$C_6$-Alkoxycarbonylgruppe substituierte niedere $C_1$-$C_6$-Alkylgruppe, eine durch eine niedere $C_1$-$C_6$-Alkylcarbonylgruppe substituierte niedere $C_1$-$C_6$-Alkylgruppe, eine Benzylgruppe (wobei eine solche Benzylgruppe durch ein Halogenatom, eine Trifluormethylgruppe, eine niedere $C_1$-$C_6$-Alkylgruppe, eine niedere $C_1$-$C_6$-Alkoxygruppe oder eine niedere $C_1$-$C_6$-Alkoxycarbonylgruppe substituiert sein kann), $R^2$ ein Wasserstoffatom oder eine niedere $C_1$-$C_6$-Alkylgruppe, $R^3$ eine niedere $C_1$-$C_6$-Alkylgruppe, eine Phenylgruppe oder eine Benzylgruppe (wobei eine solche Phenyl- oder Benzylgruppe durch ein Halogenatom, eine Trifluormethylgruppe, eine niedere $C_1$-$C_6$-Alkylgruppe, eine niedere $C_1$-$C_6$-Alkoxygruppe, eine niedere $C_1$-$C_6$-Alkoxycarbonylgruppe oder eine Nitrogruppe substituiert sein kann), oder $R^2$ und $R^3$ zusammen mit dem Stickstoffatom, an das sie gebunden sind, eine gesättigte heterocyclische Gruppe mit 5-7 Gliedern bilden; n = 1 ist; X ein Sauerstoffatom oder Schwefelatom; und

G =

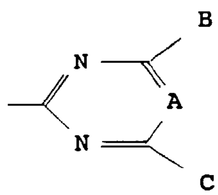

ist, worin A eine CH-Gruppe oder ein Stickstoffatom ist und B und C jeweils unabhängig voneinander eine niedere $C_1$-$C_4$-Alkylgruppe, eine niedere $C_1$-$C_4$-Alkoxygruppe, eine niedere $C_1$-$C_4$-Halogenalkylgruppe, eine niedere $C_1$-$C_4$-Halogenalkoxygruppe, ein Halogenatom oder eine niedere $C_1$-$C_4$-Mono-Alkylaminogruppe ist.

2. Die Verbindung gemäß Anspruch 1, worin $R^1$ eine $C_2H_5$-Gruppe, $R^2$ eine $CH_3$-Gruppe, $R^3$ eine $CH_3$-Gruppe, A eine CH-Gruppe, B eine $CH_3O$-Gruppe und C eine $CH_3O$-Gruppe ist.

3. Die Verbindung gemäß Anspruch 1, worin $R^1$ eine $CH_3$-Gruppe, $R^2$ eine $CH_3$-Gruppe, $R^3$ eine $CH_3$-Gruppe, A eine CH-Gruppe, B eine $CH_3O$-Gruppe und C eine $CH_3O$-Gruppe ist.

4. Ein selektives Herbizid, welches eine oder mehrere der in den Ansprüchen 1 bis 3 definierten Verbindungen als aktive Inhaltsstoffe enthält.

5. Eine Herbizid-Zusammensetzung, welche eine oder mehrere der in den Ansprüchen 1 bis 3 definierten Verbindungen und ein aus der aus Alachlor, Acetochlor, Metolachlor, Primisulfuron und Nicosulfuron bestehenden Gruppe ausgewähltes Mitglied als aktive Inhaltsstoffe enthält.

6. Eine Sulfamidosulfonylisocyanat-Verbindung der Formel (II):

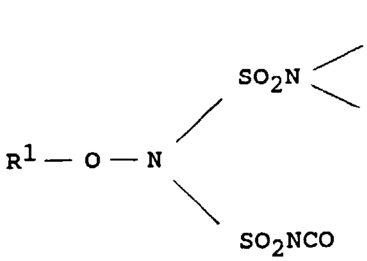

(II)

worin $R^1$, $R^2$ und $R^3$ wie in Anspruch 1 definiert sind.

7. Eine Sulfamidosulfonamid-Verbindung der Formel (IV):

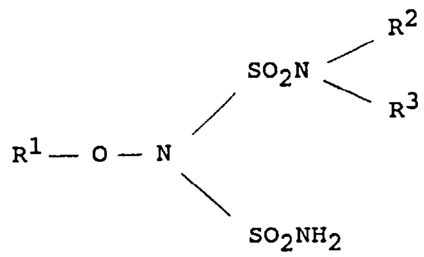

(IV)

worin $R^1$, $R^2$ und $R^3$ wie in Anspruch 1 definiert sind.

**8.** Eine Sulfamidosulfonylcarbamat-Verbindung der Formel (V):

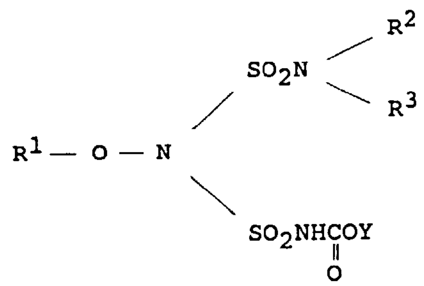

(V)

worin $R^1$, $R^2$ und $R^3$ wie in Anspruch 1 definiert sind und Y eine $C_1$-$C_6$-Alkylgruppe oder Phenylgruppe ist.

**9.** Eine N-(t-Butylsulfamoyl)sulfamid-Verbindung der Formel (XI):

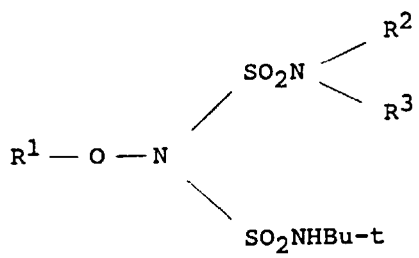

(XI)

worin $R^1$, $R^2$ und $R^3$ wie in Anspruch 1 definiert sind.

**10.** Eine Alkoxysulfamid-Verbindung der Formel (VI):

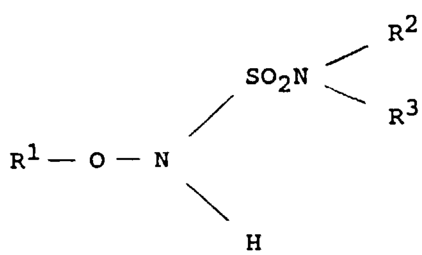

(VI)

worin $R^1$, $R^2$ und $R^3$ wie in Anspruch 1 definiert sind.

**11.** Ein Verfahren zur Herstellung einer Sulfamidosulfonylharnstoff-Verbindung der Formel (I):

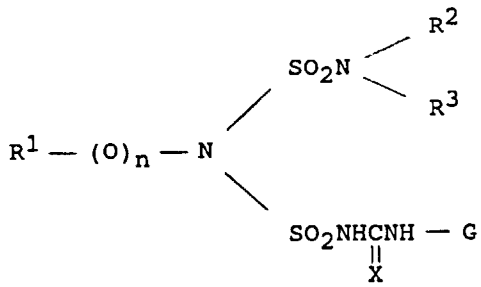

(I)

worin $R^1$, $R^2$, $R^3$ und n wie in Anspruch 1 definiert sind, welches umfaßt:

(a) die Umsetzung einer Sulfamidosulfonylisocyanat-Verbindung der Formel (II):

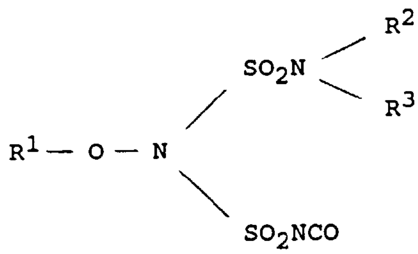

(II)

worin $R^1$, $R^2$ und $R^3$ wie oben definiert sind, mit einer Pyrimidin-Verbindung der Formel (III):

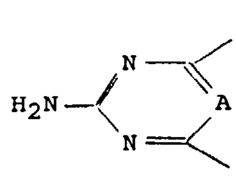

(III)

worin A, B und C wie oben definiert sind, um eine Verbindung der Formel (I) zu erhalten;
(b) die Umsetzung einer Sulfamidosulfonamid-Verbindung der Formel (IV):

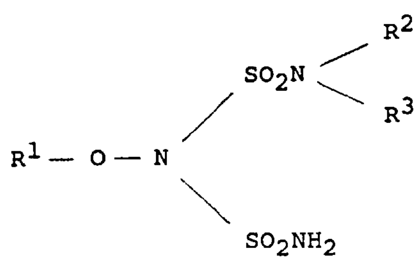

(IV)

worin $R^1$, $R^2$ und $R^3$ wie oben definiert sind, mit einer Carbamatverbindung der Formel (VIII):

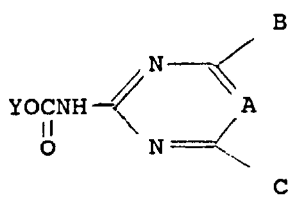

(VIII)

worin A, B und C wie oben definiert sind und Y eine $C_1$-$C_6$-Alkylgruppe oder eine Phenylgruppe ist, um eine Verbindung der Formel (I) zu erhalten;
(c) die Umsetzung einer Sulfamidosulfonylcarbamat-Verbindung der Formel (V):

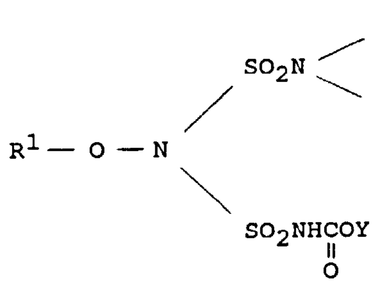

(V)

worin $R^1$, $R^2$ und $R^3$ wie oben definiert sind und Y eine $C_1$-$C_6$-Alkylgruppe oder eine Phenylgruppe ist, mit einer Pyrimidin-Verbindung der Formel (III):

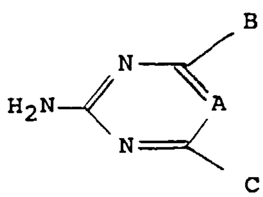

(III)

worin A, B und C wie oben definiert sind, um eine Verbindung der Formel (I) zu erhalten;
(d) die Umsetzung einer Pyrimidin-Verbindung der Formel (III):

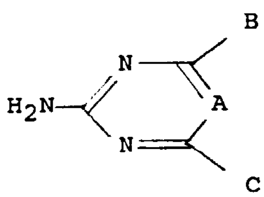

(III)

worin A, B und C wie oben definiert sind, mit Chorsulfonylisocyanat und dann mit einer Sulfamid-Verbindung der Formel (VI):

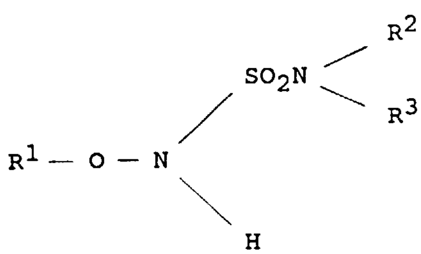

(VI)

worin $R^1$, $R^2$ und $R^3$ wie oben definiert sind, um eine Verbindung der Formel (I) zu erhalten.

## Revendications

**1.** Dérivé de sulfamidosulfonylurée de formule (I) ou sel de ce dernier approprié en agriculture :

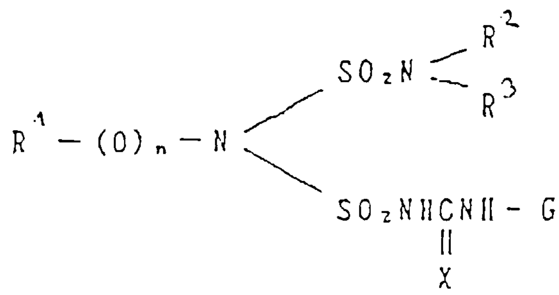

(I)

dans laquelle :

- $R^1$ représente un atome d'hydrogène, un groupe alkyle inférieur en $C_1$-$C_6$, un groupe cycloalkyle en $C_3$-$C_7$, un groupe alcényle inférieur en $C_2$-$C_6$, un groupe alcynyle inférieur en $C_2$-$C_6$, un groupe alkyle inférieur en $C_1$-$C_6$ substitué par un groupe alcoxy inférieur en $C_1$-$C_6$, un groupe alkyle inférieur en $C_1$-$C_6$ substitué par un groupe mono- ou polyhalogénoalcoxy inférieur en $C_1$-$C_6$, un groupe alkyle inférieur en $C_1$-$C_6$ substitué par un groupe alkylthio inférieur en $C_1$-$C_6$, un groupe alkyle inférieur en $C_1$-$C_6$ substitué par un groupe alkylsulfonyle inférieur en $C_1$-$C_6$, un groupe mono- ou polyhalogénoalkyle inférieur en $C_1$-$C_6$, un groupe alkyle inférieur en $C_1$-$C_6$ substitué par un groupe cyano, un groupe alkyle inférieur en $C_1$-$C_6$ substitué par un groupe (alcoxy inférieur en $C_1$-$C_6$)carbonyle, un groupe alkyle inférieur en $C_1$-$C_6$ substitué par un groupe (alkyl inférieur en $C_1$-$C_6$)carbonyle, un groupe benzyle (à la condition qu'un tel groupe benzyle puisse être substitué par un atome d'halogène, un groupe trifluorométhyle, un groupe alkyle inférieur en $C_1$-$C_6$, un groupe alcoxy inférieur en $C_1$-$C_6$ ou un groupe (alcoxy inférieur en $C_1$-$C_6$)carbonyle) ;
- $R^2$ est un atome d'hydrogène ou un groupe alkyle inférieur en $C_1$-$C_6$ ;
- $R^3$ est un groupe alkyle inférieur en $C_1$-$C_6$, un groupe phényle ou un groupe benzyle (à la condition qu'un tel groupe phényle ou un tel groupe benzyle puissent être substitués par un atome d'halogène, un groupe trifluorométhyle, un groupe alkyle inférieur en $C_1$-$C_6$, un groupe alcoxy inférieur en $C_1$-$C_6$, un groupe (alcoxy inférieur en $C_1$-$C_6$)carbonyle ou un groupe nitro) ; ou
- $R^2$ et $R^3$ forment, conjointement avec l'atome d'azote auquel ils sont liés, un groupe hétérocyclique saturé, à 5-7 chaînons ;
- n vaut 1 ;
- X est un atome d'oxygène ou un atome de soufre ; et
- G représente

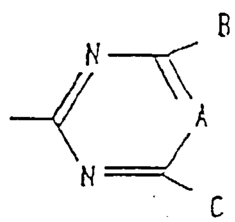

où :

- A représente un groupe CH ou un atome d'azote ; et
- B et C représentent chacun indépendamment un groupe alkyle inférieur en $C_1$-$C_4$, un groupe alcoxy inférieur en $C_1$-$C_4$, un groupe halogénoalkyle inférieur en $C_1$-$C_4$, un groupe halogénoalcoxy inférieur en $C_1$-$C_4$, un atome d'halogène ou un groupe mono-alkylamino inférieur en $C_1$-$C_4$.

**2.** Composé selon la revendication 1, dans lequel :

- $R^1$ est un groupe $C_2H_5$ ;
- $R^2$ est un groupe $CH_3$ ;
- $R^3$ est un groupe $CH_3$ ;
- A est un groupe CH ;
- B est un groupe $CH_3O$ ; et
- C est un groupe $CH_3O$.

**3.** Composé selon la revendication 1, dans lequel :

- $R^1$ est un groupe $CH_3$ ;
- $R^2$ est un groupe $CH_3$ ;
- $R^3$ est un groupe $CH_3$ ;
- A est un groupe CH ;
- B est un groupe $CH_3O$ ; et
- C est un groupe $CH_3O$.

**4.** Herbicide sélectif contenant un ou plusieurs composés définis à l'une des revendications 1 à 3 en tant qu'ingrédients actifs.

**5.** Composition herbicide comprenant un ou plusieurs composés définis à l'une des revendications 1 à 3 et un élément choisi dans le groupe constitué par l'alachlor, l'acétochlor, le métolachlor, le primisulfuron et le nicosulfuron, en tant qu'ingrédients actifs.

**6.** Composé sulfamidosulfonylisocyanate de formule (II) :

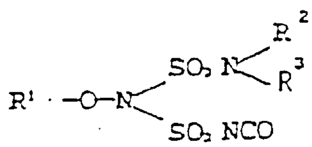

(II)

dans laquelle $R^1$, $R^2$ et $R^3$ sont tels que définis à la revendication 1.

**7.** Composé sulfamidosulfonamide de formule (IV) :

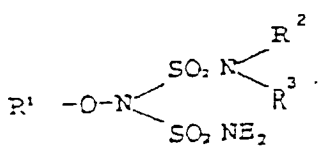

(IV)

dans laquelle $R^1$, $R^2$ et $R^3$ sont tels que définis à la revendication 1.

**8.** Composé sulfamidosulfonylcarbamate de formule (V) :

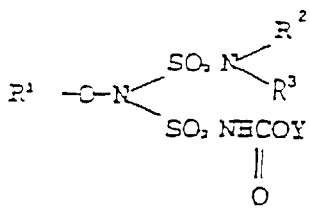

(V)

dans laquelle :

- $R^1$, $R^2$ et $R^3$ sont tels que définis à la revendication 1 ; et
- Y est un groupe alkyle en $C_1$-$C_6$ ou un groupe phényle.

**9.** Composé N-(t-butylsulfamoyl)sulfamide de formule (XI) :

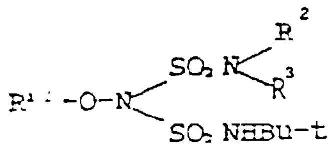

(XI)

dans laquelle $R^1$, $R^2$ et $R^3$ sont tels que définis à la revendication 1.

**10.** Composé alcoxysulfamide de formule (VI) :

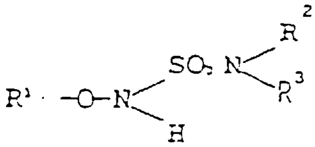

(VI)

dans laquelle $R^1$, $R^2$ et $R^3$ sont tels que définis à la revendication 1.

**11.** Procédé de fabrication d'un composé sulfamidosulfonylurée de formule (I) :

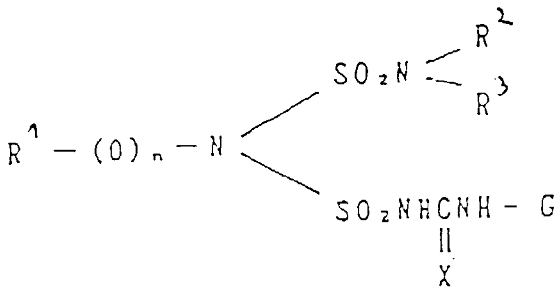

(I)

dans laquelle $R^1$, $R^2$, $R^3$ et n sont tels que définis à la revendication 1,
qui comprend les opérations consistant à :

(a) faire réagir un composé sulfamidosulfonylisocyanate de formule (II) :

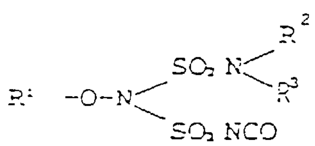

(II)

dans laquelle $R^1$, $R^2$ et $R^3$ sont tels que définis ci-dessus,
avec un composé pyrimidine de formule (III) :

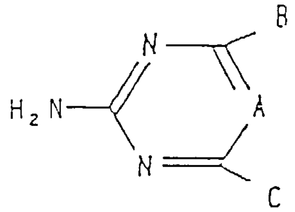

(III)

dans laquelle A, B et C sont tels que définis ci-dessus,

afin d'obtenir un composé de formule (I) ;

(b) faire réagir un composé sulfamidosulfonamide de formule (IV) :

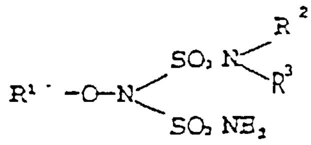

(IV)

dans laquelle $R^1$, $R^2$ et $R^3$ sont tels que définis ci-dessus, avec un composé carbamate de formule (VIII) :

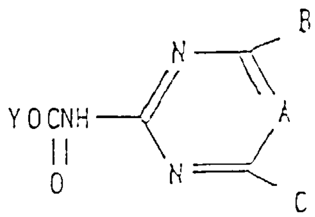

(VIII)

dans laquelle :

- A, B et C sont tels que définis ci-dessus ; et
- Y est un groupe alkyle en $C_1$-$C_6$ ou un groupe phényle,

afin d'obtenir un composé de formule (I) ;

(c) faire réagir un composé sulfamidosulfonylcarbamate de formule (V) :

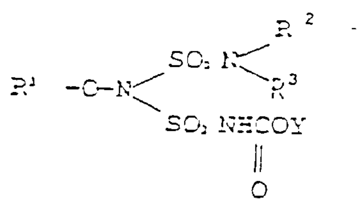

(V)

dans laquelle :

- $R^1$, $R^2$ et $R^3$ sont tels que définis ci-dessus ; et
- Y est un groupe alkyle en $C_1$-$C_6$ ou un groupe phényle,

avec un composé pyrimidine de formule (III) :

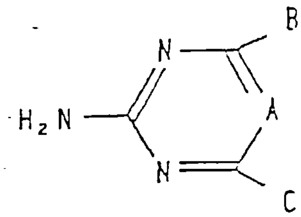

(III)

dans laquelle A, B et C sont tels que définis ci-dessus, afin d'obtenir un composé de formule (I) ;

(d) faire réagir un composé pyrimidine de formule (III) :

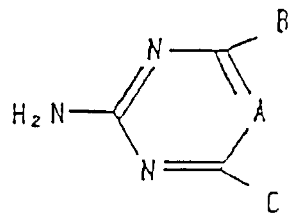

(III)

dans laquelle A, B et C sont tels que définis ci-dessus,
avec un chlorosulfonylisocyanate, puis faire réagir avec un composé sulfamide de formule (VI) :

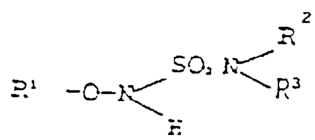

(VI)

dans laquelle $R^1$, $R^2$ et $R^3$ sont tels que définis ci-dessus,
afin d'obtenir un composé de formule (I).